# EUROPÄISCHE PATENTSCHRIFT

(11) **EP 4 030 991 B1**
(45) Veröffentlichungstag und Bekanntmachung des Hinweises auf die Patenterteilung: **25.06.2025**
(21) Anmeldenummer: 20786243.4
(22) Anmeldetag: 15.09.2020
(51) Int. Cl.: A61B 5/053

(54) **ANALYSENSYSTEM UMFASSEND EINE ZAHNBÜRSTE**
ANALYSIS SYSTEM COMPRISING A TOOTHBRUSH
SYSTÈME D'ANALYSE COMPRENANT UNE BROSSE À DENTS

(30) Priorität: 16.09.2019 DE 202019105110 U
(43) Veröffentlichungstag der Anmeldung: 27.07.2022
(73) Patentinhaber: Bioinitials GmbH, 4105 Biel-Benken, BL (CH)
(72) Erfinder: SCHÄFER, Christoph, 6390 Engelberg (CH); CIRILLO, Fabio, 4466 Ormalingen (CH); JÄGGI, Christian, 4105 Biel-Benken (CH); SCHAFFNER, Stefan, 4142 Münchenstein (CH); STÜBINGER, Stefan, 4102 Binningen (CH); JEZERNIK, Saso, 8057 Zürich (CH)
(74) Vertreter: Kleine, Hubertus
(86) Internationale Anmeldenummer: PCT/EP2020/075780
(87) Internationale Veröffentlichungsnummer: WO 2021/052970

(56) Entgegenhaltungen:
- DE-T5- 112012 003 494
- US-A1- 2009 317 770
- US-A1- 2012 322 023
- US-A1- 2014 310 900
- US-A1- 2016 374 609

## Beschreibung

Die vorliegende Erfindung betrifft ein Analysesystem umfassend eine Zahnbürste (100) mit einem Bürstenkopf gemäss Anspruch 1.

Die US 6,623,698 B offenbart eine Vorrichtung zur Detektion von Blutzucker, Schwangerschaft, HIV und dergleichen. Diese Zahnbürste geht von einem und/oder mehreren wechselbaren Zahnbürstenaufsätzen aus. Die Aufsätze weisen integrierte Sensoren auf.

Die Sensoren sind entweder optische Sensoren oder elektrische Sensoren. Die Druckschrift offenbart eine zeitsequentielle Messung mit einer ersten Zeitphase, in welcher eine Speichelsammlung und -messung erfolgt, und eine anschließende zweiten Zeitphase, in der nur ein Bürsten der Zähne erfolgt.

Obgleich diese Druckschrift und somit auch die damit verbundene Technologie bereits seit geraumer Zeit bekannt ist, hat sich ein damit-realisiertes Produkt nicht kommerziell durchgesetzt. Ein möglicher Grund könnte in der Art der Sensoren der Bürste gesehen werden. Bei Einsatz von Biosensoren mit geringer Lebensdauer von mehreren Tagen in einem vorgenannten Bürstenkopf wären die Austauschintervalle des Bürstenkopfes sehr gering.

Weitere relevante Dokumente zum Stand der Technik sind die US 2012/322 023 A1, die DE 11 2012 003 494 T5, die US 2016/374609 A1 und die US 2014/310900 A1.

Andererseits muss in der Zahnmedizin besonders auf Hygiene geachtet werden.

Weiterhin sind manche Sensoren nach kurzem, meist sogar einmaligem Gebrauch nicht mehr verlässlich.

Ausgehend davon ist es die Aufgabe der vorliegenden Erfindung eine Zahnbürste bereitzustellen, welche einen Bürstenkopf mit integrierter und austauschbarer Sensoreinheit aufweist.

Die vorliegende Erfindung löst diese Aufgabe durch ein Analysesystem mit den Merkmalen des Anspruchs 1.

Ein Bürstenkopf einer Zahnbürste weist eines oder mehrere Mittel zur mechanischen Reinigung der Zähne auf. Diese werden nachfolgend in einer bevorzugten Ausführungsform als eine Mehrzahl an Borsten zur Reinigung der Zähne beschrieben. Typischerweise sind diese Borsten entlang einer Vorderseite angeordnet.

Es ist aber auch im Rahmen der vorliegenden Erfindung möglich, dass alternativ oder zusätzlich zu den besagten Borsten, eines oder mehrere andere Mittel zur mechanischen Reinigung von Zähnen eingesetzt werden. Dieses oder diese Mittel können u.a. einen Schwamm, ein Kissen, eine oder mehrere Mikrofaserstrukturen oder eines oder mehrere Vibrationselemente umfassen. Entsprechend handelt es sich bei der Bürste mit Mikrofaserstrukturen sodann um eine Textilbürste mit Textil statt Borsten oder um eine Schwammbürste mit einer Schwammstruktur statt Borsten oder eine Vibrationsbürste mit einem oder mehreren Vibrationselementen.

Nachfolgend erfolgt die weitere Beschreibung anhand der bevorzugten Ausführungsvariante mit den Borsten, wobei selbstverständlich auch andere Mittel, wie beschrieben eingesetzt werden können.

Weiterhin weist der Bürstenkopf eine Sensoranordnung auf, welche der Überwachung des Gesundheitszustandes eines Anwenders dient. Als Gesundheit wird im Rahmen der vorliegenden Erfindung sowohl die Anwesenheit und Konzentration von Krankheitserregern, wie Viren, Pilzen und/oder Bakterien, verstanden als auch die Anwesenheit von Karzinomen, kardiovaskuläre Krankheiten, Magengeschwüren, Reflux, Diabetes oder auch der Fertilitätszustand, z.B. bevorstehender Eisprung, oder auch der Schwangerschaftszustand eines Anwenders. Die vorgenannte Aufzählung ist nicht abschließend, sondern kann durch weitere Beispiele ergänzt werden.

Die Überwachung des Gesundheitszustandes kann u.a. durch Messung von Krankheitserregern wie z.B. Konzentration von Bakterien, Viren u. dergleichen erbracht werden oder z.B. auch durch den Nachweis von Abwehrreaktionen des Körpers, wie z.B. den Nachweis von Antikörpern, erbracht werden. Auch andere Messungen, wie z.B. Temperatur und/oder Leitfähigkeit, können allein oder in Kombination mit weiteren Messdaten zur Überwachung des Gesundheitszustandes genutzt werden.

Die Überwachung des Gesundheitszustands kann optional und bevorzugt anhand eines personalisierten Gesundheitsprofils erfolgen, welches im gesunden Zustand, z.B. einem Arztbesuch, erstellt wurde. Die Änderungen der Messungen können in diesem Fall stets mit diesem Gesundheitsprofil verglichen werden.

Im Unterschied zum bisherigen Stand der Technik ist die Sensoreinheit austauschbar am oder im Bürstenkopf der Zahnbürste angeordnet. Die Sensoreinheit kann Teil einer Sensoranordnung sein, welche ebenfalls austauschbar ausgebildet sein kann.

Der Bürstenkopf selbst kann austauschbar am Griff einer Zahnbürste angeordnet sein oder aber fest mit der Zahnbürste verbunden sein. Typischerweise ist der Austauschzeitraum eines Bürstenkopfes nach mehr als 1 Monat, beispielsweise alle 3-4 Monate, während die Sensoreinheit oder die Sensoranordnung vorzugsweise nach jeder Messung oder nach wenigen Messungen ausgetauscht werden sollte.

Die Sensoranordnung weist mehrere unterschiedliche Sensoreinheiten auf.

Bei Austausch der Sensoranordnung können somit mehrere Sensoreinheiten zeitgleich ausgetauscht werden. Wiederum andere Sensoreinheiten können fest, also nicht austauschbar, mit dem Bürstenkopf und/oder dem Griff der Zahnbürste verbunden sein.

Die Sensoreinheit kann auf der Seite des Bürstenkopfes, auf der Rückseite oder auch zwischen den Borsten, analog zu einem Sieb angeordnet sein.

Die Nutzung einer austauschbaren Wegwerf-Sensoreinheit (disposable sensor unit) und besonders bevorzugt einer austauschbaren Wegwerf-Sensoranordnung (disposable sensor arrangement) ermöglicht beispielsweise eine einmalige und dosierte Abgabe eines Indikators, z.B. eines Indikatorfarbstoffs oder Biomarkers, an eine bzw. die vorgenannte Sensoreinheit. Gleiches gilt auch für die pH-Wertmessung. Danach kann die Sensoreinheit oder die Sensoranordnung ausgetauscht werden. Die Sensoreinheit und/oder die Sensoranordnung muss daher nicht für den Wiedergebrauch besonders robust ausgebildet sein, sondern kann in miniaturisierter Ausführung realisiert werden.

Für den Fall, dass Reagenzien oder die Sensoreinheit als Ganzes bei der Messung verbraucht und/oder zerstört wird, ist eine Austauschbarkeit besonders vorteilhaft.

Vorteilhafte Ausgestaltungen des Bürstenkopfes sind Gegenstand der Unteransprüche.

Vorteilhaft kann der Bürstenkopf einen sogenannten Sensor-Adapter, insbesondere einen Ein-, Zwei-, oder Drei-Wege-Adapter (three-way connector) zur Verknüpfung der austauschbaren Sensoreinheit oder der austauschbaren Sensoranordnung, z.B. eines Biosensors, mit dem Bürstenkopf aufweisen.

Der Bürstenkopf kann bevorzugt eine elektrische Schnittstelle aufweisen mit Kontakten, insbesondere in Form von kugelförmigen, vorzugsweise vergoldeten, Kontaktelementen, welche als Matrix angeordnet sind. Die Matrix kann eine lineare 1-dimensionale Anordnung aufweisen oder als 2-dimensionale Matrix ausgebildet sein. Vorzugsweise ist die Matrix abgedichtet gegenüber Flüssigkeiten, einschließlich von Saliva bzw. Speichel mit entsprechender IP-Klasse, z.B. IP67. Bevorzugt sind sogenannte BGA-Kontakte (Ball-Grid-Array bzw. Ball-Gitter-Struktur) als Kontaktelemente vorgesehen. Zusätzlich oder alternativ zu den vorgenannten BGA-Kontakten können auch Pins und/oder Lamellen vorgesehen sein. Insgesamt können somit mehrere Konnektor- /Interface-Kontakte, vorzugsweise in Form von BGA-Kontakten, Pins und/oder Lamellen, vorgesehen sein.

Der Bürstenkopf, insbesondere die Sensoreinheit oder die Sensoranordnung, kann zumindest ein Reservoir und/oder eine Zuleitung für Abgabestoffe, insbesondere von Biomarkern, z.B. funktionale Moleküle, Bindestoffe, z.B. Bindeliganden, und/oder Indikatorverbindungen, z.B. Luminophore, insbesondere Fluorophore, aufweisen. Im Fall von Bindeliganden können diese mit anderen Molekülen im Speichel und mit der Sensoroberfläche eine Bindung herstellen. Entsprechende Ligand-Rezeptor Wechselwirkungen und die entsprechenden Bindemolekülen sind aus dem Anwendungsbereich von ELISA-Tests (enzyme-linked immunosorbent assays) bekannt und können im Rahmen der vorliegenden Erfindung auch als Bindeliganden eingesetzt werden.

Es ist von Vorteil, wenn die Sensoreinheit, oder besonders bevorzugt die Sensoranordnung umfassend mehrere Sensoreinheiten, als Dünnschicht- oder Dünnfilm-Sensoreinheit oder -anordnung oder als Sensorchip ausgebildet ist. Dadurch wird eine Miniaturisierung der Sensoranordnung erreicht und der Bürstenkopf muss nur wenig Platz für die Sensoranordnung oder die Sensoreinheit bereitstellen. Alternativ oder zusätzlich kann die Sensoranordnung mittels eines Mikrotechnologieverfahrens additiv und/oder subtraktiv ausgebildet werden, so z.B. mittels Lithographie, Soft-Lithographie, 3D-Printing, Bedruckung, Siebdruck und/oder Stereolithographie gefertigt sein.

Die Sensoranordnung kann vorteilhaft zumindest zwei, vorzugsweise zumindest drei, Sensoreinheiten zur Ermittlung von zumindest zwei Stoffeigenschaften von Speichel, vorzugsweise drei Stoffeigenschaften von Speichel, insbesondere zumindest einer physikalischen Stoffeigenschaft und zumindest einer chemischen, biochemisch und/oder biologischen Stoffeigenschaft, aufweisen. Aus den ermittelten Stoffeigenschaften kann eine Tendenz zur Krankheitsausbildung abgeleitet werden.

Die Sensoranordnung kann eine erste Sensoreinheit zur Bestimmung eines spannungsäquivalenten Messwertes des Mediums bzw. des Speichels, insbesondere eines Widerstandswerts des Mediums oder eines Leitfähigkeitswertes des Mediums aufweisen. Die erste Sensoreinheit ist zur Durchführung einer Impedanzmessung des Speichels mit einer oder mehreren Frequenzen und/oder Frequenzbändern ausgebildet.

Insbesondere kann die erste Sensoreinheit einen elektrischen Impedanzwert ermitteln, wobei die Impedanzwerte vorzugsweise über mehreren Frequenzen und/oder Frequenz-Bänder bestimmt werden können. Der DC-Widerstand ist in der Impedanz als DC-Wert enthalten. Der Wirkwiderstand R ist der frequenzunabhängige, reelle Teil der komplexen Impedanz Z(f)=R+jX(f). Beschichtete Elektroden und Elektrolyten führen zu komplexen Impedanzen von Biosensoren wegen deren kapazitiven Eigenschaften (Aufladung von Elektroden, Elektrodenoberflächen, Isolationen, Permittivität von Elektrolyt / Körperflüssigkeiten). Auch eine zeitliche (dynamische) Spannungs- und/oder Strommessung und/oder eine Ladungsmessung (Coulomb) könnte man zur Bestimmung des spannungsäquivalenten Widerstandswertes nehmen. Auch eine modell-basierte Ermittlung der vorgenannten Größen kann erfolgen.

Die Sensoranordnung weist vorteilhaft zumindest eine zweite Sensoreinheit zur Bestimmung des pH-Wertes von Speichel auf.

Ebenfalls weist die Sensoranordnung eine dritte Sensoreinheit auf, wobei die dritte Sensoreinheit als Biosensor ausgebildet ist. Ein elektrischer Bürstenkopf wird in der Regel über längere Zeitperioden z.B. 30 - 80 Tage verwendet, was im Fall von Biosensoren mit beschichteten Elektroden oder auch optische Sensoren kein realistischer Anwendungszeitraum ist. Die Messungen verlieren an Genauigkeit, Stabilität, Spezifizität, Selektivität und können schon nach ein paar Tagen unbrauchbar werden.

In einer bevorzugten Ausführungsvariante der Erfindung ist der Biosensor als ein Hormon-, Enzym-, Peptid- und/oder Protein-Sensor, einschließlich einem Antigen- und/oder Antikörper-Sensor, und besonders bevorzugt als ein Lactat-Sensor, ein Glucose-Sensor, ein GABA-Sensor, ein IgA-Sensor, ein Lysozym-Sensor, ein IgG-Sensor, ein IgM-Sensor, IL-6 Sensor, CRP Sensor, Tnf-a Sensor und/oder ein alpha. Macroglobulin Sensor ausgebildet. Diese Verbindungen sind Indikatoren für Periodontitis, Paradontitis, Diabetes und/oder kardiovaskuläre Krankheiten. Alternativ oder zusätzlich kann der Biosensor als einer oder mehrere Sensoren zur Detektion von Metaboliten von Krankheiten ausgebildet sein.

Als krankheitsverursachende Bakterien können vorzugsweise von paradontitis- und/oder kariesverursachenden Bakterien, vorzugsweise von Prevotella intermedia, Porphyromonas gingivalis, Tannerella forsythia, Treponema denticola und den mit aggressiver Parodontitis assoziierten Aggregatibacter actinomycetemcomitans verstanden werden, welche Metaboliten erzeugen, die detektierbar sind. Allerdings können auch Metabolite anderer Krankheiten, beispielsweise von Viren wie SARS-Covid oder Grippeviren sowie andere Krankheitserreger von Erkältungen oder Mundracheencarcoinomen gemessen werden. Auch diverse Krankheitserreger wie beispielsweise Bakterien, Viren sowie Fungi, insbesondere Schimmel, sind detektierbar.

Bevorzugt können durch die dritte Sensoreinheit oder durch weitere Sensoreinheiten verschiedene Metaboliten oder Analyten pro Krankheitsbild detektiert werden, um eine Fehlmessung (ein sogenanntes "false-negative") auszuschließen.

Die Sensoranordnung und/oder eine einzelne Sensoreinheit kann form- oder kraftschlüssig auf der borstenabgewandten Seite des Bürstenkopfes angeordnet sein, beispielsweise durch Verklemmen, Verrasten und/oder Verpressen.

Weiterhin kann eine mechanische und/oder elektrische Schnittstelle, z.B. unter Einbeziehung des vorgenannten Adapters, vorgesehen sein. An der Stelle kann ein Verriegelungs-Mechanismus zwischen der Sensoranordnung und/oder der Sensoreinheit und dem Bürstenkopf vorgesehen sein, so dass ein Austausch nur nach Betätigung des Verschluss-Mechanismus möglich ist.

Bei der Signalverarbeitung können bevorzugt auch Raw-Signale bzw. Rohdaten mit einer genügend hohen Abtastung verarbeitet werden - vorzugsweise im Bereich 10 bis 1000 Hz und über längere Zeitperioden, vorzugsweise von zumindest einer Minute. Die Zeitperiode kann auch mehr als 4 Minuten betragen und die Abtastung kann in Abhängigkeit vom Zustand von Zahnbürste erfolgen. Wird z. B. eine Abtastung während einer Ultraschallwellenbehandlung durch eine elektrische Zahnbürste vorgenommen, so kann im Ruhezustand ein anderer Zeitraum gewählt werden als im Modus, in dem die Vibrationen aktiv sind (um den Störeinfluss von Vibrationen auf die SU Messung zu vermeiden). Im Falle von Abtastung während aktiven Vibrationen können durch avancierte Signalverarbeitung (DSP) auch Methoden angewendet werden, die die Störungen rausfiltern oder kompensieren, so dass eine Stör -Aufschaltung bzw. Kompensation erfolgt. Dazu können Störeinflüsse geschätzt oder ermittelt werden und die Signale wie in einem Feedforward-Disturbance Compensation Schema (bekannt aus der Regelungstechnik) oder adaptiven Noise-Canceller (bekannt aus DSP - Digital Signal Processing: Adaptive Noise Canceller von Widrow, Echo-Cancellers, adaptive LMS Filter) Schema kompensiert und/oder adaptiert werden.

Die austauschbare Sensoreinheit mit Biosensoren wird elektrochemische Signale laut den entwickelten Redox Reaktionen, die vorzugsweise spezifisch für die Target-Biomarker Biosensoren entwickelt worden sind, liefern. Da diese Einheit Messwerte, vorzugsweise über mehrere Messungen und/oder mehrere Tage, auf eine zuverlässige Art und Weise liefern sollte (mit geeigneter Präzision und Stabilität), sind fortschrittliche Signalverarbeitungsmethoden von Vorteil.

Eine Vielzahl der derzeitigen elektrochemischen Biosensoren müssen normalerweise vor der Messung in einer geeigneten Lösung vorkonditioniert werden, über Zeitintervalle von 10-30 Minuten.

Alternativ oder zusätzlich kann die Sensoranordnung auch elektro-optische Sensoren aufweisen, mittels denen eine Änderung der Fluoreszenz oder eine spektrale Änderung oder eine Änderung des refraktiven Indexes (RI-Index/Brechungsindex) detektierbar ist.

Bei der vorliegenden Anwendung können Vorkonditionierungen in den Anwendungs-Zyklen verkürzt werden oder sie können vorteilhaft entfallen. Eine Vorkonditionierung kann leichter bei der Vorrichtung mit Austausch-Station realisierbar sein, aber schwieriger ohne eine solche Austauschstation, also bei einer elektronischen Zahnbürste ohne eine entsprechende Austausch- oder Ladestation, also als alleinstehende SU-Einheit. Man kann aber jedoch die Vorkonditionierung ebenfalls vorteilhaft umgehen, indem man eine oder eine Kombination mehrerer DSP Methoden anwendet. Diese können auch für die weitere Funktionalität vom Produkt benutzt werden, wie Detektion vom Zustand von Biosensoren - trocken vs. mit Speichel benetzt, Detektion von guter Signalqualität vs. schlechter Signalqualität z.B. auch Entdeckung von zwischenzeitlichen Zuständen, bei denen die SU-Einheit nicht ausreichend mit Speichel benetzt ist, oder Bewegungen detektiert werden, die Störungen verursachen.

Weiterhin Teil der Erfindung ist eine Zahnbürste.

Die Zahnbürste kann vorteilhaft eine Steuer- und/oder Auswerteeinheit aufweisen, welche ausgerüstet ist zum Erfassen von Daten, insbesondere von Rohdaten, während der Messung des Speichels durch die Sensoreinheit mit einer Abtastrate von 10-1000 Hz.

Die Steuer- und/oder Auswerteeinheit kann zudem ausgerüstet sein zum Erfassen von Daten, insbesondere von Rohdaten, während der Messung des Speichels durch die Sensoreinheit in einem Abtastintervall von zumindest 3 bis 500 Sekunden.

Weiterhin weist die Zahnbürste ein Funkmodul zur Datenübertragung mit einem externen Gerät zur Datenverarbeitung unter Ermittlung eines Gesundheitszustandes auf.

Das Funkmodul kann Teil eines Elektronikmoduls sein, welches zudem ein Messmodul umfassen kann. Das Elektronikmodul kann eine oder mehrere ASIC's als anwendungsspezifische integrierte Schaltungen beinhalten.

Zusätzlich kann die Zahnbürste ein Modul zur induktiven Leistungsübertragung, welches vorzugsweise im Griff der Zahnbürste angeordnet ist, aufweisen.

Weiterhin ist ein Analysensystem vorgesehen, umfassend die vorbeschriebene Zahnbürste und ein externes Gerät zur Datenverarbeitung, wobei Rohsignale von der Zahnbürste auf das externe Gerät übermittelt werden und wobei das externe Gerät einen Datenspeicher aufweist auf welchem ein Computerprogrammprodukt zur Datenanalyse hinterlegt ist, wobei das Computerprogrammprodukt ausgebildet zur Datenanalyse der von der Sensoreinheit ermittelten Daten, vorzugsweise nach einer oder mehreren DSP-Methode (Methode der digitalen Datenverarbeitung bzw. digital signal processing), zur Ermittlung eines Gesundheitszustandes.

Ein Verfahren zum Betrieb des erfindungsgemäßen Analysensystems umfasst eine Datenanalyse und weist die folgenden Schritte auf:
a) Abtasten von elektrochemischen Signalen, insbesondere von amperometrischen Signalen bei unterschiedlichen Potenzialen und/oder voltametrischen Signalen bei unterschiedlichen Strömen, und/oder Impedanzsignalen über eine Messdauer mit einer vorbestimmten Abtastrate

Die bevorzugte Messdauer und Abtastrate wurden bereits zuvor beschrieben.
b) Detektion und Klassifizierung von Zuständen des Sensorelements hinsichtlich des "Befeuchtungszustandes" anhand der ermittelten elektrochemischen Signalen und/oder Impedanzen

In einer bevorzugten Ausführungsvariante kann die vorgenannte Detektion durch eine Impedanzmessung erfolgen. In einem Frequenzbereich zwischen 500 Hz bis 100 kHz wurden folgende Werte detektiert. Die Werte können mit der Frequenz variieren.
Speichel: typischer Bereich: 0.6-1 kOhm
Speichel mit Wasser: 1-1.6 kOhm
ausschliesslich Wasser wird viel höhere Werte aufzeigen, da nicht so gut leitend. Es werden Werte von 3-8 kOhm für Leitungswasser angenommen
Zahnpasta im Speichel reduziert die Impedanzwerte auf 0.2-0.5 kOhm; die Reduktionen sind abhängig von Zusammensetzung der Zahnpasta und der verwendeten Menge, je mehr Zahnpasta desto stärker die Reduktion der Impedanzwerte.

Somit kann man die vorbeschriebene Impedanzmessung verwenden, um die folgenden Zustände zu detektieren: Wasser über Sensoren (vollständige oder teilweise Befeuchtung der Sensoren), Wasser-Speichel-Mix, Speichel, Speichel mit Zahnpasta
c) Bewertung von Amplituden und Änderungen der elektrochemischen Signale und/oder Impedanzen und insbesondere der damit einhergehenden Redox-Reaktionen und/oder kapazitiven Transienten um den Speichel hinsichtlich des Gesundheitszustands des Anwenders zu analysieren

Die Bewertung kann insbesondere durch einen Istwert- und Sollwertvergleich durch eine Auswerteeinheit erfolgen, wobei vordefinierte Datensätze für die Bewertung auf einem Datenspeicher der Auswerteeinheit hinterlegt sind, z.B. in sogenannten Lookup Tabellen.

Es ist insbesondere von Vorteil, wenn in Schritt c) eine Zerlegung und/oder Filterung der abgetasteten elektrochemischen Signale in eine Nutzkomponente und eine oder mehrere Störkomponenten des Signals erfolgt, wobei der Nutzanteil die Redoxreaktionskomponente des Signals repräsentiert bzw. mit dieser korreliert, welche für die Konzentrationsbestimmung von Biomarker relevant ist. Die Korrelation kann beispielsweise linear oder exponentiell oder dergleichen sein.

Das Verfahren, insbesondere im Anschluss an Schritt c) oder vor Schritt a), kann zumindest einen der weiteren Schritte aufweist:
d) Abtastung von elektrochemischen Signalen nach einer Konditionierung und/oder Stabilisierung in einer bereitgestellten Referenzlösung; und/oder
e) Wiederholungen von Messungen, um die Information aus mehreren Messungen zu kombinieren, vorzugsweise um die Präzision zu verbessern, das Signal-zu-Rauschen (S/N signal-to-noise) Verhältnis zu verbessern und/oder Störungen zu kompensieren.

Weiterhin kann eine Austauschstation für den Austausch des Bürstenkopfes vorgesehen werden mit einer Austauschkammer, wobei der Bürstenkopf zumindest bereichsweise in die Austauschkammer einführbar ist und wobei die Austauschstation eine Vorratsanordnung mit mehreren vereinzelbaren Sensoranordnungen und/oder Sensoreinheiten und eine Bestückungseinheit zum Bestücken des Bürstenkopfes innerhalb der Austauschkammer aufweist. Die Austauschkammer ist zumindest zu einer Seite hin geöffnet, um den Bürstenkopf einzuführen.

Das Bestücken kann beispielsweise automatisch oder durch manuelle Krafteinwirkung erfolgen. Als Austauschstation kommen viele Varianten in Betracht. So kann die Austauschstation beispielsweise entweder eine Größe eines Tischaufsatzes oder einer kleinen von Hand-betriebenen Station aufweisen. Insbesondere in letzteren Fall kann die Station mit einer Batterie oder einem anderen portablen Stromspeicher, z.B. einem Akku, betrieben werden.

Weiterhin vorteilhaft kann die Austauschstation ein Funkmodul, z.B. eine wireless Sende- und Empfangseinheit, aufweisen, zum bidirektionalen kabellosen Datenaustausch, insbesondere mit einem externen Rechner oder Server oder einer Cloudanwendung.

Eine Austauschstation kann optional Sensoren aufweisen, welche durch den Bürstenkopf gesammelte Speichelproben selbst auswertet. Hierfür kann die Austauschstation eine oder mehrere Fluidleitungen zum Spülen eines Messraums, in welchen die Speichelprobe eingeführt wird, aufweisen, sowie eine oder mehrere Fluidleitungen zur Zugabe von Reagenzien in den Messraum, sowie eine Mess- und Auswerteeinheit, welche die von den Sensoren der Austauschstation erfassten Messwerte auswerten. Der Messraum kann zugleich auch die Austauschkammer sein. Über einen Mechanismus, vorzugsweise einen Mechanismus wie vorbeschrieben, kann die Zahnbürste, insbesondere deren Borsten, oder deren Kissen, Schwamm oder anderes Material auch einen Wirkstoff abgeben.

Ein weiteres Konzept der vorliegenden Offenbarung ist, dass die Station keine Sensoreinheiten oder Sensoranordnungen austauscht, sondern als Lagerstation lediglich eine Auswertung von Speichel betreibt, welcher durch die Zahnbürste gesammelt wurde. Diese Sammlung von Speichel erfolgt über einen Einsaugmechanismus innerhalb der Zahnbürste. Dieser Speichel wird sodann an den Messraum abgegeben und innerhalb des Messraumes durch hierfür angeordnete zumindest teilweise austauschbare Sensoren ausgewertet.

Die Austauschstation kann vorteilhaft eine Ladestation, z.B. eine Ladeanschlussstelle, aufweisen, zum Aufladen der Zahnbürste.

Weiterhin kann die Austauschstation eine Auffang- und/oder Auswerteeinheit aufweisen, zum Auslesen von Sensordaten der verbrauchten Sensoranordnung und/oder zur Kalibration oder zum Einlesen von Sensordaten für die zu bestückende Sensoranordnung.

Es ist von Vorteil, wenn die Austauschstation und die Zahnbürste zumindest ein Sendemodul, insbesondere ein Sende- und Empfangsmodul, zum Datenaustausch von ermittelten Sensordaten mit einer externen Datenverarbeitungseinheit aufweist. Hier kommt beispielsweise ein Bluetooth-Modul, ein NFC-Modul, ein RFID-Modul, ein LoRa-Modul, ein 5G-Modul oder dergleichen in Betracht.

Im Fall des Sende- und Empfangsmoduls oder eines zusätzlichen Empfangsmoduls kann ein bidirektionaler Datenaustausch erfolgen.

Die Austauschstation kann ein Modul für einen kabellosen Energietransfer, z.B. durch induktive Leistungsübertragung, aufweisen. Dieses Modul kann auch eine Datenübertragung vornehmen, so dass das Modul vorteilhaft auch die Funktion eines Sende- und/oder Empfangsmodul einnehmen kann. Ein Modul für kabellosen Energie-Transfer kann auch zusätzlich im Zahnbürstengriff und/oder im Zahnbürstenaufsatz enthalten sein.

Es können somit diverse Daten von der Zahnbürste, der Sensoranordnung oder der jeweiligen austauschbaren Sensoreinheit ausgelesen werden. Dies umfasst z.B. Kalibrationsdaten, UDI-Daten (also eine Seriennummer oder andere Daten zur Identifikation der Zahnbürste und/oder der Sensoranordnung und/oder der Sensoreinheit), Konfigurationsdaten und selbstverständlich auch Sensordaten, z.B. Messwerte oder Messbedingungen. Weiter bevorzugt können QAM, FSK oder PSK für die Datenübertragung verwendet werden.

Der Datenaustausch erfolgt vorzugsweise bidirektional zwischen der Austauschstation und dem Bürstenkopf. Hierfür kann der Bürstenkopf oder die Zahnbürste einen Mikrocontroller vorzugsweise mit EEprom (elektrisch löschbaren, programmierbaren Nur-Lese-Speicher) aufweisen. Zudem kann der Bürstenkopf oder die Zahnbürste einen anderen nicht-volatilen RAM-Speicher aufweisen. Dieser Speicher kann in einem optionalen ASIC-Chip, separat im Mikrocontroller oder in weiteren Elektronikbauteilen des Bürstenkopfes oder der Zahnbürste angeordnet sein.

Die Austauschstation kann weitere optionale Einheiten aufweisen, welche die Analysevarianten und/oder die Bedienbarkeit der Zahnbürste erweitern. Weiter können Messbedingungen sowie Zustände der Elektronik bzw. Gerätezustände eingestellt und überprüft werden. Beispielsweise kann die Austauschstation eine Erkennungseinheit aufweisen, welche die Identität der Zahnbürste bzw. des damit verbundenen Benutzers erkennt und die Einstellung individuell an diese Person vornimmt. Hierfür weist die Zahnbürste eine Markierung, z.B. einen QR-Code oder dergleichen, auf.

Eine in der Austauschstation oder in der Zahnbürste vorgesehene Auswerteeinheit kann zur Ausführung eines Verfahrens zum Betrieb der Zahnbürste zur Überwachung des Gesundheitszustandes, insbesondere mit Hinblick auf die Bildung von Paradontitis, bei einem Anwender bzw. Patienten ausgebildet sein, wobei das Verfahren die folgenden Schritte umfasst:
I. Bestücken des Bürstenkopfes der Zahnbürste mit einer neuen Sensoranordnung und/oder Sensoreinheit, insbesondere in der Austauschstation;
II. Einführen des Bürstenkopfes in den Mundraum und Kontaktierung der Sensoranordnung und/oder Sensoreinheit mit Speichel;
   Hierdurch wird die Sensoranordnung und/oder Sensoreinheit verbraucht. Das Verbrauchen kann insbesondere bereits nach einmaligem Kontaktieren eintreten.
III. Erzeugung von Sensordaten zur Ermittlung von zumindest zwei, vorzugsweise zumindest drei, Stoffeigenschaften des Speichels und
IV. Entnahme einer verbrauchten Sensoranordnung und/oder Sensoreinheit und Auslesen der Daten, insbesondere innerhalb der Austauschstation.

Einige der Schritte, z.B. Schritt **III** und IV, können zeitgleich erfolgen. In Schritt I kann zudem optional und vorteilhaft eine Kalibration der Sensoreinheiten erfolgen.

Selbstverständlich kann auch lediglich eine oder mehrere Stoffeigenschaften als Summe ermittelt werden, wobei die Stoffeigenschaften Summenparameter sein können, welche modellbasiert ermittelbar sind. Es kann auch lediglich die Änderung der Stoffeigenschaften ermittelt werden. Auch dies fällt unter den vorgenannten Schritt III. Weiterhin können weitere Biomarker externer Geräte verarbeitet werden und insbesondere in den Summenparameter eingehen. Ein typisches Beispiel hierfür sind die Daten des Programms Applehealth Vital Signs.

Nach dem Ausführen des Schrittes II des Verfahrens kann ein Anzeigemodul angeben, dass die Sensoranordnung verbraucht ist und zur Wiederholung des Verfahrens ausgetauscht werden muss. Der Einsatz des Anzeigemoduls ist allerdings bei Ausführung des Verfahrens nicht zwingend notwendig. Die Anzeige kann z.B. akustisch, optisch (z.B. durch eine LED-Anzeige oder ein Display z.B. ein Display auf dem Gerät selber oder auf einem Mobilgerät, wie z.B. einem Smartphone) oder durch Vibrationen erfolgen.

Weiterhin kann eine Vorrichtung zur Überwachung des Gesundheitszustandes, insbesondere mit Hinblick auf die Bildung von Paradontitis, Periodontitis, Diabetes oder kardiovaskulären Erkrankungen bei einem Anwender vorgesehen werden, umfassend eine Zahnbürste mit einer Vorrichtung zum Entnahme von Speichel eines Patienten und eine Station mit einer Messkammer zur Aufnahme des angesaugten Speichels, wobei innerhalb der Messkammer zumindest eine Sensoreinheit oder eine Sensoranordnung umfassend zumindest eine Sensoreinheit angeordnet ist und wobei die Sensoreinheit oder die Sensoranordnung austauschbar innerhalb der Messkammer angeordnet sind. In diesem Fall ist die Station keine Austauschstation zum Austausch der Sensoranordnung oder Sensoreinheit am Bürstenkopf, sondern die Sensoreinheit oder Sensoranordnung ist innerhalb der Messkammer bzw. Austauschkammer angeordnet. Für die Kontaktierung mit dem Speichel des Patienten wird in diesem Fall die Zahnbürste nur als Transportmittel genutzt. Die Erzeugung von Messdaten erfolgt somit nicht im Mund des Patienten, sondern erst innerhalb der Station.

Es ist bei diesem Konzept besonders von Vorteil, wenn die Messkammer einen Vorrat, insbesondere ein Magazin, an unverbrauchten Sensoreinheiten oder Sensoranordnungen aufweist und einen Austauschmechanismus, z.B. eine Entnahme- und Bestückungsmaschine, zum Austausch einer verbrauchten Sensoreinheit oder Sensoranordnung gegen eine neue Sensoreinheit oder Sensoranordnung aus dem Vorrat bzw. dem Magazin aufweist.

Die Vorrichtung zur Entnahme des Speichels in der Zahnbürste kann vorzugsweise als eine Ansaugvorrichtung ausgebildet ist und beispielsweise eine Ansaugpumpe und/oder Abgabepumpe umfassen, z.B. zum Transport und der Verabreichung von Flüssigkeiten.

In einer vorteilhaften Variante des Verfahrens ist nach dem Ausführen des Schrittes II des Verfahrens die Sensoranordnung verbraucht und muss vorzugsweise zur Wiederholung des Verfahrens ausgetauscht werden. Diese Variante wird meist als "single-use" bzw. Einmalgebrauch beschrieben.

Weiterhin dient die Verwendung des eBürstenkopfes zur Überwachung des Gesundheitszustandes, insbesondere der Konzentration an Viren, Bakterien im Körper, des Entzündungszustandes im Körper, des Diabetes- und/oder Karzinomrisikos, des Risikos einer kardiovaskulären Erkrankung und/oder des Fertilitätszyklusses, eines Anwenders.

Nachfolgend wird die Erfindung im Detail anhand eines Ausführungsbeispiels unter Zuhilfenahme von Figuren näher erläutert. Abwandlungen des Ausführungsbeispiels werden ebenfalls genannt und können als isolierte Merkmale verstanden werden.
- Fig. 1: schematische Darstellung eines Bürstenkopfes einer Zahnbürste;
- Fig. 2: schematische Darstellung einer Anordnung umfassend eine Austauschstation und den Bürstenkopf der Fig. 1;
- Fig. 3: schematische Darstellung einer zweiten Ausführungsvariante eines Bürstenkopfes;
- Fig. 4: eine weitere Ansicht des Bürstenkopfes der Fig. 3;
- Fig. 5: schematische Darstellung einer dritten Ausführungsvariante eines Bürstenkopfes;
- Fig. 6: vergrößerte Ansicht der Fig. 5;
- Fig. 7: schematische Darstellung eines Ausschnitts einer vierten Ausführungsvariante in Abwandlung der Variante der Fig. 5 und 6
- Fig. 8: Komplettdarstellung der Variante der Fig. 7;
- Fig. 9: schematische Darstellung einer fünften Ausführungsvariante eines Bürstenkopfes;
- Fig. 10: weitere Ansicht des Bürstenkopfes der Fig. 9;
- Fig. 11: vereinfachte Explosionsansicht der Variante der Fig. 9 und 10;
- Fig. 12: schematische Darstellung einer sechsten Ausführungsvariante eines Segments eines Bürstenkopfes.

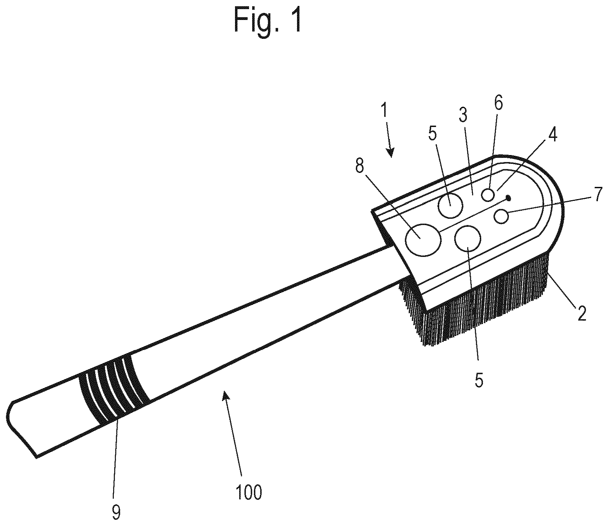

Fig. 1 zeigt einen Bürstenkopf 1 einer Zahnbürste 100. Im vorliegenden Fall handelt es sich um den Bürstenkopf 1 einer elektrischen Zahnbürste. Es kann sich selbstverständlich auch um eine Zahnbürste ohne elektrischen Antrieb zur Bewegung der Bürsten und/oder ohne Ultraschallgeber handeln. In diesem Fall wird zwar eine Energieversorgungseinheit benötigt, welche allerdings nur die Sensoreinheiten, insbesondere die Bio- und Elektrosensorik, ggf. Sende- und/oder Empfangsmodule und ggf. eine Auswerteeinheit betreibt.

Der Bürstenkopf 1 weist vorderseitig eine Mehrzahl von Borsten 2 auf und rückseitig eine austauschbare Sensoranordnung 3 umfassend mehrere Sensoreinheiten. Die Sensoranordnung 3 ist als flexibler Kunststofffilm oder als Chip auf einem Kunststoffsubstrat ausgebildet und umfasst zumindest eine erste Sensoreinheit 4 zur Bestimmung des pH-Wertes des Messmediums. Dabei handelt es sich vorzugsweise um eine Wegwerf-Sensoreinheit, insbesondere für den Einmal-Gebrauch. Die Sensoreinheit kann insbesondere als Dünnschicht- und/oder Dünnfilm-Sensoreinheit oder als eine andere Form einer Sensoreinheit auf Basis von Mikrotechnologie, z.B. als MEMS-Einheit, ausgebildet sein. Weitere Varianten zur Ausbildung einer entsprechenden Sensoreinheit sind z.B. 3D-Printing, Tampondruck, Lithographie oder Siebdruck.

Weiterhin kann der Bürstenkopf 1 einen Sensor-Adapter, insbesondere einen Ein-, Zwei-, oder Drei-Wege-Adapter zur Verknüpfung der austauschbaren Sensoranordnung 3 aufweisen, welcher allerdings in Fig. 1 unterhalb der Sensoranordnung und damit verdeckt angeordnet ist.

Typische Dünnfilm-Sensoreinheiten zur Messung des pH-Wertes sind beispielsweise Kohlenstoffelektroden dotiert mit Ruthenium-Dioxid. Derartige pH-Elektroden werden als Wegwerf-Sensoren für den Einmal-Gebrauch z.B. von Koncki et al, in "Disposable screen-printed pH-electrode for determination of anticholinesterase activity" (S.139 ff. Biosensors for Direct Monitoring of Environmental Pollutants in Field) beschrieben.

Auch andere pH-Dünnschichtsensoren mit pH-Elektroden basierend auf ISFET-Technologie sind an sich aus verschiedenen Anwendungen bekannt. Ein beispielhafter Aufbau eines pH-Sensors mit einer Referenzelektrode und einer Zusatzelektrode wird beispielsweise in der DE 10 2011 085 747 A1 beschrieben.

Auch die AT186493 A, die AT 184692 A und die DE 10 2006 022 290 offenbaren pH-Sensoren basierend auf Dünnschicht-Technologie oder Dickschicht-Technologie mit Elektrodenschichten, insbesondere leitenden und halbleitenden Schichten verschiedenen Materialien oder aber Sensorchips realisierbar sind.

Die Ausgestaltung der ersten Sensoreinheit ist nicht zwingend auf die dargestellten zwei Elektroden beschränkt. So kann die Sensoreinheit bevorzugt beispielsweise als Potentiostat-Anordnung mit einer Arbeitselektrode, einer Referenzelektrode und einer Gegenelektrode ausgebildet sein.

Auch der Einsatz einer sogenannten 3-Elektrodenzelle ist im Rahmen der vorliegenden Erfindung möglich, wobei in einer besonders bevorzugten Ausführungsvariante eine vierte und ggf. weitere Elektroden in den Sensoreinheiten zur Störgrößenerfassung und Störgrößen-Kompensation eingesetzt werden können. In einem von einer Auswerteeinheit durchgeführten Signalverarbeitungsverfahren wie Störungskompensationen zur Rauschverringerung, erfolgen.

Die vorgenannte Sensoreinheit bzw. die vorgenannten pH-Sensoren und die dazugehörigen elektrischen Leitungen und Anschlüsse können vorzugsweise auf oder in einer Flexprint-Leiterplatte oder einer flexiblen Folie angeordnet sein.

Als Messbereich des pH-Sensors empfiehlt sich für die Anwendung ein pH-Wert zwischen 1-13, vorzugsweise zwischen 3-8. Speichel ist bekanntermaßen neutral bis schwach basisch, so dass der vorgenannte Messbereich ideal für die Anwendung ist. Es ist zudem von Vorteil, wenn die Sensoreinheit 4 selbst einen nicht-näher dargestellten Temperatursensor aufweist, zur Ermittlung der Mediumstemperatur. Sofern diese z.B. aufgrund von Eis, Tee oder dergleichen außerhalb eines vorbestimmten Messbereichs, insbesondere zwischen 33 bis 37,5 °C liegt, kann wahlweise eine Fehlerausgabe für die Messung mit dem Hinweis erfolgen, dass die Temperatur im Rachenraum außerhalb eines vorbestimmten Spezifikationsbereichs liegt oder es kann eine Anzeige einer physiologischen Änderung, z.B. Schwangerschaft, Krankheit oder Reaktion, e.g. Immunreaktion, aufgrund erhöhter Temperatur oder Fieber ab 38,5°C sein. Die Temperatur-Messung über Tage kann auch vorteilhaft für die Überwachung des Gesundheitszustandes inklusive eines personalisierten Profils und/oder der Bestimmung vom Fertilitäts-Zyklus, z.B. zur Verhütung oder zur Fruchtbarkeitsbestimmung, sein.

Die maximale Erstreckung der vorgenannten Sensoreinheit in einer Raumrichtung kann vorzugsweise zwischen 4-8 mm betragen. Bevorzugt kann die Sensoreinheit eine Raumabmessung von weniger als 150 mm², besonders bevorzugt zwischen 50-120 mm², aufweisen.

Die Dicke der Sensoreinheit kann weniger als 4 mm, bevorzugt 2 mm oder weniger. betragen. Besonders bevorzugt können die jeweiligen Sensoren auf einer Folie als Substrat angeordnet, insbesondere aufgedruckt sein.

Die Sensoreinheit, als Folie kann vorzugsweise nach maximal 15-fachen Gebrauch, vorzugsweise maximal 10-fachen Gebrauch, gewechselt werden.

Die einzelnen Sensoren einer Sensoreinheit können rund ausgeführt sein, wobei zumindest einer oder einige der Sensoren mit einem Durchmesser zwischen 1-3 mm ausgeführt sein können.

Eine in der Zahnbürste (im Bürstenkopf oder im Bürstengriff) integrierte nichtdargestellte Steuer- und/oder Auswerteeinheit kann ausgerüstet sein zum Erfassen von Daten, insbesondere von Rohdaten, während der Messung des Speichels durch die Sensoreinheit 4, 5, 8 in einem Abtastintervall von zumindest 3 bis 500 Sekunden. Eine Messdauer von weniger als 20 Sekunden ist allerdings vorteilhaft, beispielsweise 5 Sekunden.

Weiterhin umfasst die Sensoranordnung 3 eine zweite Sensoreinheit 5, vorzugsweise eine Dünnschicht- oder Dünnfilm-Sensoreinheit, zur Bestimmung einer Spannung oder einer spannungsäquivalenten Messgröße. Dabei umfasst das zweite Dünnfilm-Sensoreinheit ein Elektrodenpaar aus zwei Elektroden 6 und 7 zur Ermittlung besagter Messgröße, so z.B. der Leitfähigkeit des Messmediums, der Impedanz des Messmediums, der Stromstärke oder auch der Spannung, bzw. eines jeweiligen Abfalls zwischen den Elektroden. Besonders bevorzugt ist die Impedanzmessung bei einer oder mehreren Frequenzen, beispielsweise Frequenzbereiche, 0.1 bis 100, von 100 bis 5000 Hz, und von 1 kHz bis 10 kHz, ggf. noch darüber.

Anstelle der zwei Elektroden 6 und 7 kann als Sensoreinheit 5 auch ein Potentiostat oder eine 3-Elektrodenanordnung vorgesehen sein. Eine Impedanzmessung kann beispielsweise zur Messung von Biomarkern wie TNF-a oder anderen Biomarkern eingesetzt werden.

Auch diese zweite Sensoreinheit 5 ist in geringen Abstand von weniger 1 cm, vorzugsweise weniger als 5 mm, besonders bevorzugt weniger als 3 mm von der ersten Sensoreinheit 4 beabstandet. Da sich die Zusammensetzung des Mediums im Mundraum ändern kann ist dieser geringe Abstand bevorzugt, so dass die Sensoreinheiten im wesentlichen Speichel gleicher Zusammensetzung analysieren.

Die Sensoranordnung 3 weist zudem eine dritte Sensoreinheit 8 auf. Die dritte Sensoreinheit 8 umfasst eine Messzelle und eine Referenzzelle zur Ermittlung einer Stoffkonzentration einer chemischen Verbindung im Speichel. Als chemische Verbindung kann z.B. der Laktat- oder Glukosegehalt ermittelt werden. Der Messbereich eines Glukose-Sensors liegt zwischen 0.1 bis 1 mM. Der Messbereich eines Laktat-Sensors liegt zwischen 0.025 bis 0.5 mM.

Weitere Varianten von Sensoren für die dritte Sensoreinheit 8 sind u.a. EnzymSensoren, Peptid-Sensoren, Protein-Sensoren oder Sensoren zur Detektion von Zellen.

Es kommt als dritte Sensoreinheit insbesondere die Ausgestaltung als ein sogenannter GABA-Sensor (engl. Gamma-Aminobutyrat-Acid), ein IgA (Immunglobulin A), IgG, IgM, CRP und/oder ein Lysozym-Sensor in Betracht. Diese dritte Sensoreinheit ermöglicht es durch Ermittlung der Konzentration zumindest einer chemischen Verbindung das Vorliegen anderer Krankheiten, welche die Messung des ersten und zweiten Sensoreinheit stören würden, zu erkennen. Ein typischer Sensor für die Ermittlung der vorgenannten Größen ist z.B. ein elektrochemischer Sensor, ein optischer Sensor oder ein oder mehrere geeignete beschichtete Biosensoren.

Der Bürstenkopf erlaubt zusätzlich eine Ermittlung des Vorhandenseins und der Konzentration von anaeroben und/oder aeroben Bakterien oder einzelner Klassen von den vorgenannten Bakterien z.B. durch Konzentrationsmessung von deren Abbauprodukten und/oder von Metaboliten und erlaubt eine Bewertung des Zustands des Immunsystems. Durch geeignete Kombination der obengenannten Sensoren und Erfassung resp. nicht-Erfassung von Bakterien-Metaboliten kann auf eine Infektion durch Viren geschlossen werden. Dies vor allem dann, wenn Infektionswerte resp. Infektionskriterien erfüllt und detektiert werden, jedoch die Bakterien-Metaboliten nicht detektiert werden.

In der bevorzugten Ausführungsvariante der Fig. 1 weist der Bürstenkopf 1 eine mechanische Schnittstelle 9 zur lösbaren Verbindung mit einem Griff 101, insbesondere einer Antriebsvorrichtung einer elektrischen Zahnbürste 1 auf.

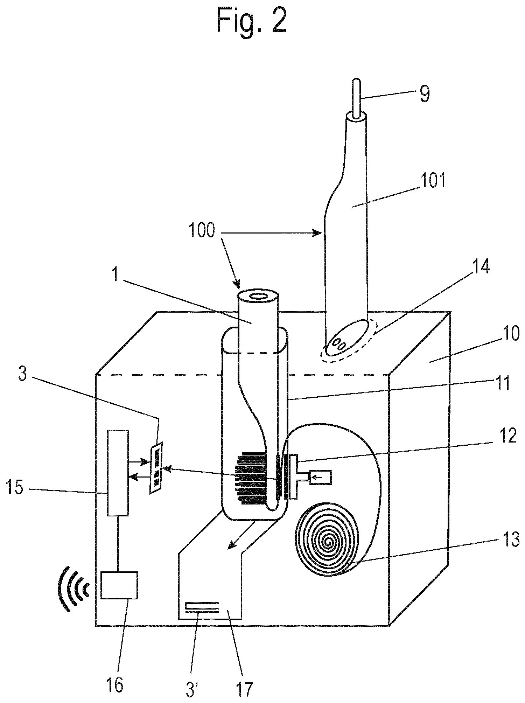

Fig. 2 zeigt eine bevorzugte Variante einer Austauschstation 10 zum Austausch der Sensoranordnung 3 auf der Rückseite des Bürstenkopfes 1. Allerdings sind auch andere Varianten von Austauschstationen 10 im Rahmen der vorliegenden Erfindung möglich.

Die Austauschstation 10 umfasst eine Austauschkammer 11, in welcher der Bürstenkopf 1 einführbar ist. In dieser Austauschkammer erfolgt eine Trennung der verbrauchten Sensoranordnung 3' und das Versehen mit einer neuen Sensoranordnung 3'. Diese neue Sensoranordnung kann als diskreter Teilabschnitt einer Vorratsanordnung 13, z.B. einer Folienrolle oder einem Magazin, durch eine Vereinzelungsvorrichtung 12 vereinzelt werden. Anschließend kann ein Bestücken des Bürstenkopfes 1 erfolgen. Im Fall der Fig. 1 ist die Vereinzelungsvorrichtung 12 vorteilhaft in kompakter Ausführung zugleich die Bestückungsvorrichtung. Es ist jedoch auch möglich, dass beide vorgenannten Vorrichtungen separat angeordnet sind. Die Bestückungsvorrichtung kann in einem ersten Schritt die verbrauchte Sensoranordnung 3' entnehmen, z.B. durch Ansaugen oder magnetische Ablösung oder dergleichen und in einem zweiten Schritt die neue Sensoranordnung 3 bestücken.

Unterhalb der Austauschkammer ist ein Reservoir 17 zur Lagerung der verbrauchten Sensoranordnungen 3 vorgesehen
Alternativ zur Folienrolle kann auch ein Magazin mit mehreren Chips in der Austauschstation 10 angeordnet sein, wobei die Chips nicht zwingend flexibel ausgebildet sein müssen.

Die Austauschstation 10 kann zudem eine integrierte Ladestation 14 aufweisen, in welchem der Griff der elektrischen Zahnbürste, insbesondere induktiv, aufgeladen werden kann.

In einer optionalen Auslese- und/oder Auswerteeinheit 15 können die Sensordaten der verbrauchten Sensoranordnung 3 ausgelesen und ggf. ausgewertet werden. Zur Speicherung der Sensordaten kann die Sensoranordnung ausgelesen werden. Anschließend können die ausgelesenen Sensordaten an eine externe Datenverarbeitungseinheit z.B. eine Datenbank eines Zahnarztes oder an einen Computer übertragen werden.

Hierfür weist die Austauschstation 10 zumindest ein Sendemodul 16, insbesondere als Sende- und Empfangsmodul, zur drahtlos-Verbindung mit der Datenverarbeitungseinheit aufweisen. Sofern das Sendemodul 16 als Sende- und Empfangsmodul ausgebildet ist oder zusätzlich zum Sendemodul 16 ein Empfangsmodul vorgesehen ist, so kann auch eine Software-Aktualisierung der Auswerteeinheit 15 erfolgen. Geeignete Technologien für die Datenübertragung sind beispielsweise 5G, LoRA, BT, NFC, über einen Hub oder ein mobiles Gerät oder direkt über eine Basisstation (LoRA, oder 5G oder 4G / cat-M, LTE Cat M1)

Es ist selbstverständlich auch möglich, dass die von der Sensoranordnung 3 ermittelten Daten nicht erst in der Austauschstation 10 übermittelt werden, sondern bereits eine Datenübertragung durch die Zahnbürste 100 erfolgt.

Die Austauschkammer 11 kann zur Reinigung mit einem Reinigungsmedium und/oder mit Wasser geflutet werden, so dass der Bürstenkopf gereinigt werden kann. Sofern die Austauschkammer 11 zugleich als Messkammer genutzt wird, kann eine Spülung zur Herstellung von möglichst gleichbleibenden Messbedingungen erfolgen. Eine Addition von Reagenzien in die Messkammer kann zudem erfolgen.

Sofern die Zahnbürste 100 über eine Versorgung mit einem Zahnputz- oder Mundspülmedium verfügt, kann die Austauschstation 10 einen Vorratsraum für besagtes Zahnputz- oder Mundspülmedium aufweisen und dieses der Zahnbürste 100 z.B. über die Ladestation 14 für eine Befüllung der Zahnbürste 100 zuleiten.

Alternativ kann auch der Auftrag an Zahnputzmittel auf die Borsten der Zahnbürste durch die Austauschstation direkt erfolgen.

Weiterhin kann die Austauschkammer 11 oder eine Kammer, welche fluidmechanisch mit der Austauschkammer 11 verbunden ist, eine Sensoranordnung z.B. für eine optische Messung aufweisen. Zur Überführung in die zusätzliche Kammer kann optional eine Absaugvorrichtung vorgesehen sein.

Der am Bürstenkopf 2 anhaftende Speichel kann auf ein vordefiniertes Volumen, z.B. das Kammervolumen, verdünnt und/oder begrenzt werden und anschließend optisch, elektrochemisch oder physikalisch vermessen werden. Hierfür kann eine Zuleitung für Abgabestoffe, z.B. funktionalen Molekülen, Bindestoffe, z.B. Bindeliganden, und/oder Indikatorverbindungen, z.B. Luminophore, insbesondere Fluorophore, vorgesehen sein, eine Lichtquelle zum Aussenden eines Lichts zur Anregung der Indikatorverbindung mit zumindest einer Wellenlänge und eine Empfangseinheit zur Messung des empfangenen Lichts. Entsprechend kann eine Fluoreszenzspektroskopische Untersuchung, eine UV-Vis- Spektroskopische Untersuchung und weitere optische Analyseverfahren durch optische Sensorik angewandt werden.

Es kann vorkommen, dass das Zahnputz- oder Reinigungsmedium einzelne Messungen stört. Daher bietet es sich an eine Referenzmessung ohne Speichel von Zeit zu Zeit durchzuführen. Dadurch wird es der Auswerteeinheit bei Messung einer Speichelprobe nach dem Zähneputzen möglich, die Menge an verwendetem Zahnputz- oder Reinigungsmedium im Speichel zu quantifizieren und die dadurch auftretenden Störungen im Messsignal zu kompensieren. Dies kann alternativ oder zusätzlich modell-basiert auf Grundlage hinterlegter Parameter oder durch nichtparametrische modell-basierte Verfahren oder durch Populationszählung, z.B. von Bakterien, erfolgen.

Alternativ kann diese Referenzmessung auch entfallen, z.B. wenn die jeweilige Analysemethode störungsunempfindlich ist oder wenn stets das gleiche Zahnputz- oder Reinigungsmedium genutzt wird, für welches der Hersteller bereits einen Datensatz im Datenspeicher der Auswerteeinheit zur Messwertkompensation hinterlegt hat.

Die Ermittlung des allgemeinen Gesundheitszustandes und speziell des oralen Gesundheitszustandes innerhalb des Mundraums kann anwenderindividuell oder durch Abschätzung anhand eines Durchschnittswerts erfolgen. Bei ersterem Fall ist der Gesundheitszustand durch einen Arzt festzustellen und im Anschluss an die Untersuchung muss eine Referenzmessung erstellt und zum Vergleich der von der Zahnbürste ermittelten Daten hinterlegt werden.

Neben dem bevorzugten Anwendungsgebiet der Früherkennung der Paradontitis (z.B. durch Messung von IgA),weitere Zahnerkrankungen (z.B. durch Messung von GABA) oder auch Karies (z.B. durch Messung von Lactobacillus) kann die vorliegende Erfindung auch zur Früherkennung einzelner Krebserkrankungen und/oder von Schwangerschaft, z.B. durch Ermittlung des Folsäuregehalts, genutzt werden.

Weitere Anwendungsfälle der Früherkennung, welche ebenfalls im Rahmen der vorliegenden Erfindung erkannt werden können, sind Carcinome, cardiovaskuläre Anwendungen, Herzinfarkt, virale Erkrankungen, Autoimmunerkrankungen, Infektionskrankheiten, gastroösophageale Refluxkrankheit, Arteriosklerose, Ischias, Atembeschwerden, insbesondere hervorgerufen durch Atemwegserkrankungen, chronische obstruktive Lungenerkrankungen, Alzheimer, Multiple Sklerose, allg. orale Gesundheitszustände, Diabetes, Sauerstoffaufnahme, Krebs, Arthritis und/oder Fehlernährung, insbesondere Fettleibigkeit.

Neben der Früherkennung kann durch den Gegenstand der vorliegenden Anmeldung auch zur Überwachung der Ernährung in der Schwangerschaft genutzt werden. Schwangere werden aufgrund des zusätzlichen Bedarfs an Nährstoffen durch das ungeborene Kind regelmäßig auf Werte wie Eisen, Folsäure und dergleichen überprüft. Hier kann die Zusammensetzung des Speichels Auskunft darüber geben, ob der tägliche Bedarf einzelner Nährstoffe bereits erreicht wurde und erhalten bleibt.

Es ist zudem möglich die Medikation eines Patienten auf diese Weise zu überwachen. Hierfür kann z.B. eine Speichelprobe des Patienten vor der Medikation als Referenz dienen.

Eine vorteilhafte Anwendung des Bürstenkopfes umfasst eine Überwachung der Medikamenteneinnahme durch den Anwender (Patient Adherence / Patient Compliance Monitoring), z.B. bei Diabetes.

Hier sollten beispielsweise die Glucose Werte in einem Ziel-Band liegen, was in schwereren Fällen nur durch die regelmäßige Einnahme von Insulin oder GLP-1 Hemmern oder ähnlichen oralen Medikamenten, die die Blutzuckerwerte im Blut erniedrigen (GLP inhibitors wie z.B. Semaglutide von Novo Nordisk) erreicht werden kann.

Mit der zeitlichen Analyse von Zeitreihen von Biosensor Daten-Werten kann man zudem detektieren nicht nur ob, sondern auch wann, der Patient die Medikamente genommen hat. Wenn der Patient die Medikamente nicht einnimmt, dann werden andere Werte, ganz oder zum Teil außerhalb vom gewünschten Band gemessen. Das Band bzw. der Sollbereich kann zum Beispiel als Band zwischen 70 und 150 mg/dL definiert werden, was die Glukose Werte im Normallfall vor und nach dem Essen darstellt.

Die in Fig. 2 dargestellte Station ist als eine Austauschstation ausgebildet. In einer nicht-dargestellten Ausführungsvariante der Erfindung ist es allerdings auch möglich, die Sensoranordnung des Bürstenkopfes der Zahnbürste in der Station, insbesondere in einen Messraum der Station, anzuordnen. Bei diesem Konzept muss allerdings der Speichel aus dem Mundraum in die Station überführt werden. Hierfür kann die Zahnbürste z.B. eine Ansaugvorrichtung aufweisen, welche Speichel im Mundraum in einem ersten Betriebsmodus entnimmt. In einem optionalen zweiten Betriebsmodus kann die Ansaugvorrichtung eine Ausblas-Funktion aufweisen, um den angesaugten Speichel in den Messraum abzugeben und dort zu vermessen.

Weiterhin kann der Bürstenkopf oder insbesondere die Austauschstation weitere Sensoren aufweisen, welche es ermöglichen einzelne Bestandteile einer Zahnpasta, eines Mundwassers oder andere Zusammensetzungen zur oralen Pflege zu detektieren und anhand dieser Detektion eine Korrektur der Messwerte des Speichels vorzunehmen.

Die Austauschstation oder die andere Station können zudem eine Abgabe einer vorbestimmten Art und Menge einer Zusammensetzung zur oralen Pflege, z.B. zum Auftrag auf die Zahnbürste, aufweisen. Diese Zusammensetzung kann hinsichtlich der Konzentration seiner Bestandteile als Datensatz, z.B. im Datenspeicher einer Auswerteeinheit, hinterlegt sein.

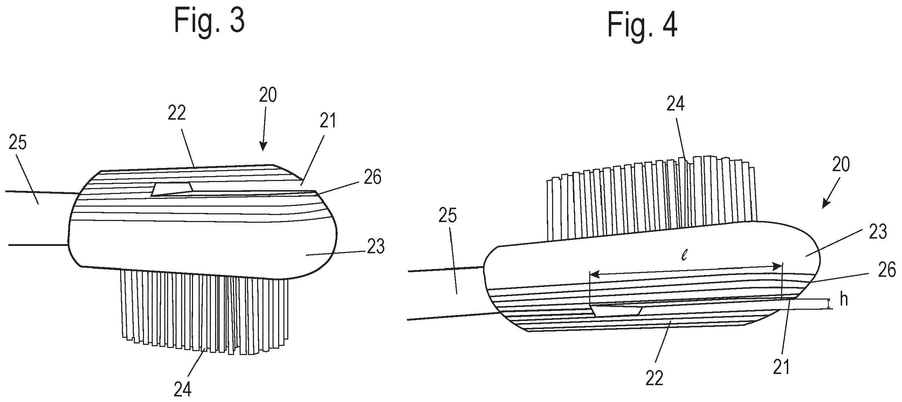

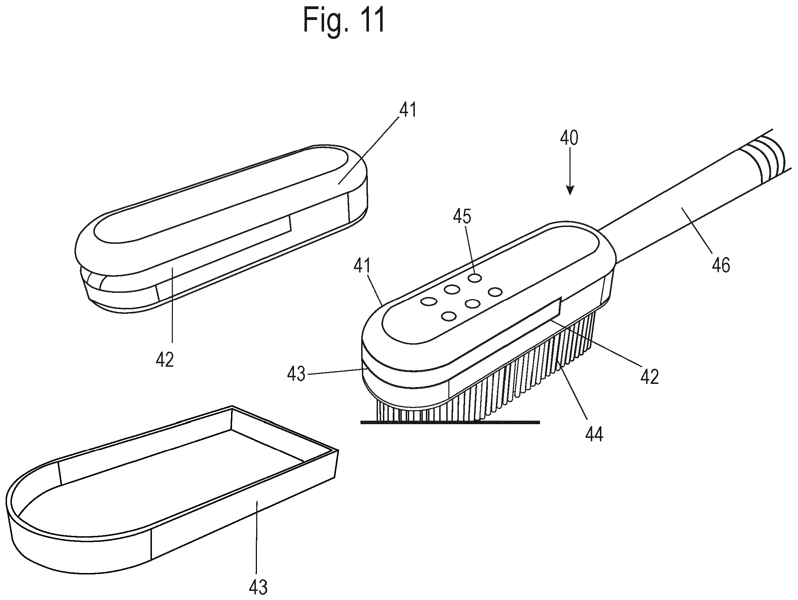

Weitere Ausführungsvarianten einer Zahnbürste sind in den Figuren 3-11 dargestellt.

Die Variante eines Zahnbürstenkopfes 20 aus Fig. 3 und 4 weist einen Grundkörper 23 auf, aus welchen Borsten 24 mit einer mittleren Borstenlänge l hervorstehen. Es können Borsten 24 verschiedenen Borstenlängen vorgesehen sein, wobei sich die mittlere Borstenlänge auf den Mittelwert aller Borsten bezieht.

Aus Gründen einer besseren Einführbarkeit in den Mund kann der Grundkörper 23 eine geringere Höhe aufweisen als die Borstenlänge l_{b}. Die Borstenlänge l_{b} und die Höhe des Grundkörpers 23 addieren sich zu einer Gesamthöhe des Bürstenkopfes.

Weiterhin weist die der Grundkörper 23 einen Einführungsschlitz 21 zur Aufnahme einer plattenförmigen Sensoreinheit auf. Die plattenförmige Sensoreinheit ist dabei den zwei Hauptflächen her vor mechanische Schädigung durch den Grundkörper 23 geschützt. Hierfür weist der Grundkörper 23 ausgehend von der Position der Borsten 24 oberseitig ein Abdecksegment 22 auf. Die Sensoreinheit ist an einer Stirnfläche 26 der Zahnbürste 20 in den Einführungsschlitz 21 einführbar, wobei die besagte Stirnfläche an einer zu einem Bürstenhals-Ansatz 25 entgegengesetzten Seite der Zahnbürste angeordnet ist.

Die Länge l des Einführungsschlitzes 21 in Einsteckrichtung R beträgt dabei vorzugsweise zumindest 30 %, besonders bevorzugt zumindest 50 %, der Länge des Bürstenkopfes in Einsteckrichtung.

Die Höhe h des Einführungsschlitzes 21 beträgt vorzugsweise zwischen 3 und 30 % der Höhe des Grundkörpers 23.

Die Variante einer Zahnbürste 20 aus Fig. 3 und 4 weist einen Grundkörper 23 auf, aus welchen Borsten 24 mit einer mittleren Borstenlänge l hervorstehen. Es können Borsten 24 verschiedenen Borstenlängen vorgesehen sein, wobei sich die mittlere Borstenlänge auf den Mittelwert aller Borsten bezieht.

Aus Gründen einer besseren Einführbarkeit in den Mund kann der Grundkörper 23 eine geringere Höhe aufweisen als die Borstenlänge l_{b}. Die Borstenlänge l_{b} und die Höhe des Grundkörpers 23 addieren sich zu einer Gesamthöhe des Bürstenkopfes.

Weiterhin weist die der Grundkörper 23 einen Einführungsschlitz 21 zur Aufnahme einer Sensoranordnung in der Ausbildung als plattenförmige austauschbare Sensoreinheit auf. Die plattenförmige Sensoreinheit ist dabei den zwei Hauptflächen her vor mechanische Schädigung durch den Grundkörper 23 geschützt. Hierfür weist der Grundkörper 23 ausgehend von der Position der Borsten 24 oberseitig ein Abdecksegment 22 auf. Die Sensoreinheit ist an einer Stirnfläche 26 der Zahnbürste 20 in den Einführungsschlitz 21 einführbar, wobei die besagte Stirnfläche an einer zu einem Bürstenhals-Ansatz 25 entgegengesetzten Seite der Zahnbürste angeordnet ist.

Die Länge l des Einführungsschlitzes 21 in Einsteckrichtung R beträgt dabei vorzugsweise zumindest 30 %, besonders bevorzugt zumindest 50 %, der Länge des Bürstenkopfes in Einsteckrichtung.

Die Höhe h des Einführungsschlitzes 21 beträgt vorzugsweise zwischen 3 und 30 % der Höhe des Grundkörpers 23.

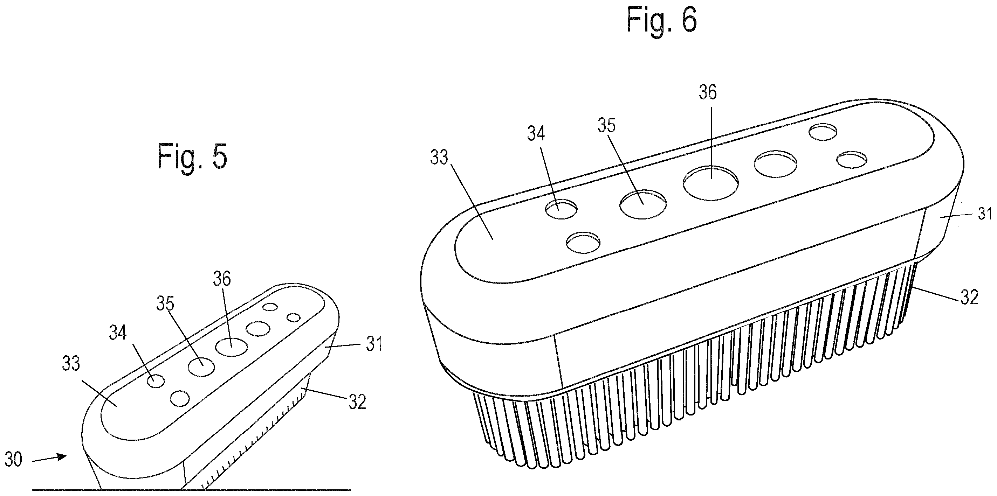

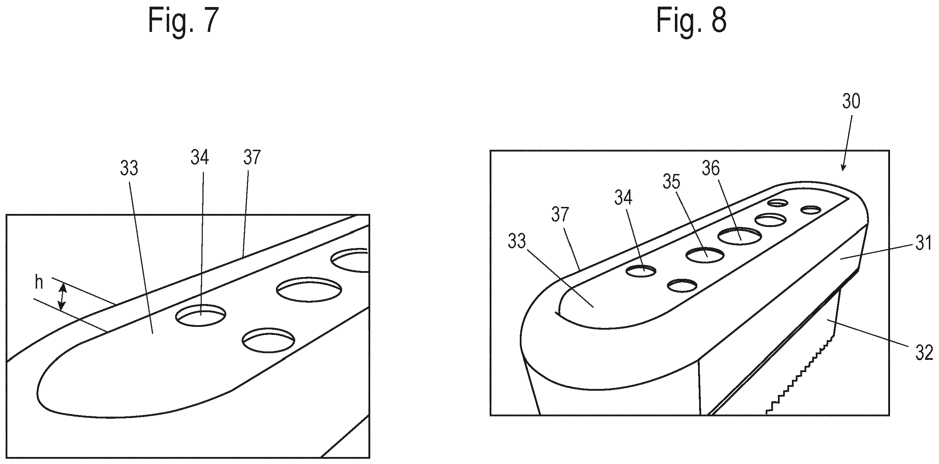

In einer weiteren Variante der vorliegenden Erfindung in Fig. 5 und 6 weist ein Bürstenkopf 30 ebenfalls einen Grundkörper 31, sowie daran hervorstehende Borsten 32 auf. Die den Borsten entgegengesetzte rückseitige Oberfläche des Bürstenkopfes weist ein Auflagenfläche 33 und darin eingelassene Sensoraufnahmen 34, 35 und 36 auf. Diese Aufnahmen ermöglichen ein Eindringen und eine Positionierung des Speichels während der Messung - was von Vorteil ist bei einem zu kurzen Speichelkontakt. Der Speichel kann in die Aufnahmen gelangen und dort in Kontakt mit den spezifischen Sensoren treten und gehalten werden,
Für eine zusätzliche verbesserte Halterung der Sensoreinheit kann ein teilweise oder vollständig umlaufender Rand 37 seitlich der Auflagenfläche 33 und aus dieser hervorstehend angeordnet sein, so dass die Sensoreinheit nicht aus der Zahnbürste hervorsteht und der Sensoreinheit beim Einsetzen eine Führung und Festlegung ermöglicht. Diese Löffelvariante ist in Fig. 7 und 8 dargestellt.

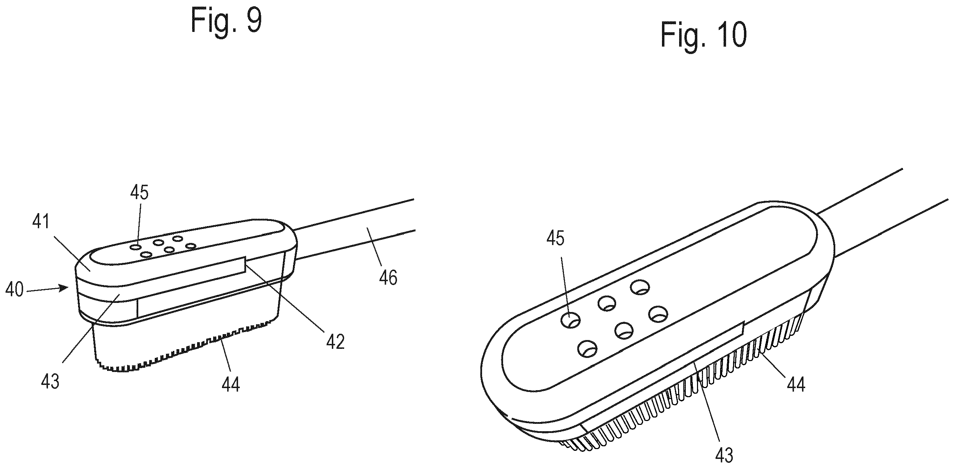

Fig. 9-11 weist eine weitere Variante eines Zahnbürstenkopfes 40 auf, welche in Analogie zur Fig. 3 und 4 einen Grundkörper 41, einen Einführungsschlitz 43 zum Einführen einer Sensoreinheit 42, sowie eine Abdecksegment 47, Im Unterschied zur Variante der Fig. 3 und 4 weist das Abdecksegment 47 Öffnungen 45 zur Einlagerung von Speichel auf. Der Speichel kann dabei durch die Öffnungen 45 durchtreten und zu den jeweiligen Sensorelementen der Sensoreinheit 43 zugeleitet werden.

Die Abdeckung kann abnehmbar oder weg-drehbar sein, um eine Reinigung und Trocknung der Biosensor-Oberfläche mit Wasser und unter Luft zu ermöglichen.

Weitere in den Figuren dargestellte Bauteile und Merkmale des Zahnbürstenkopfes sind funktional analog zur Fig. 3 und 4 zu verstehen.

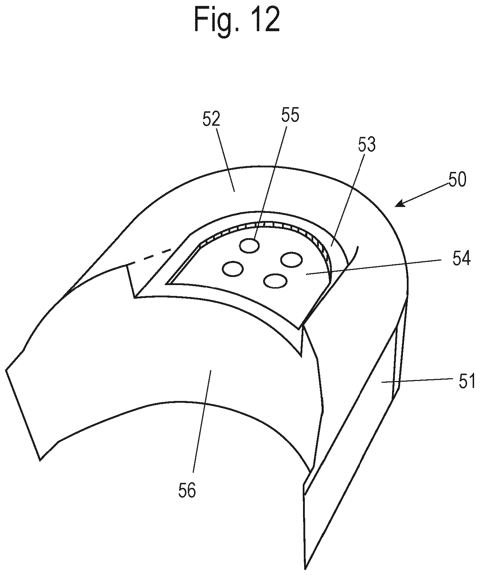

Während die Sensoreinheit in Fig. 3-11 flach ausgebildet ist, zeigt Fig 12 eine Variante eines Bürstenkopfes 50 mit einer Sensoreinheit 51 als seitlicher Aufsatz auf den Grundkörper eines Bürstenkopfes. Die Sensoreinheit 51 kann Teil einer größeren Sensoranordnung sein, wobei lediglich die Sensoreinheit 51 austauschbar ist und die anderen Sensoren der Sensoranordnung nicht-austauschbar im Bürstenkopf angeordnet sein können. Solche nicht-austauschbaren Sensoren können z.B. ein Temperatursensor oder ein Leitfähigkeitssensor, Impedanzsensoren, potentiometrische Sensoren, usw. sein. Die austauschbaren Sensoren 55 sind im Körper der Sensoreinheit 51 eingelassen, welcher randseitig zum Sensorarray 53 eine Anschlagsfläche 56 für die Adaption an den Grundkörper aufweist. Auch in dieser Variante ist eine Vertiefung 54 zum besseren Ansammeln von Speichel gegeben, sowie ein randseitiger Vorsprung 52 zu gleichem Zweck.

Alternativ ist es auch möglich einen platten oder folienartigen auswechselbaren Einsatz als Sensoreinheit in die Vertiefung 54 einzusetzen.

Die Merkmale einzelner Ausführungsvarianten können vorteilhaft miteinander kombiniert werden. Dabei ist die Anordnung der Sensoreinheit auf einer gegenüber dem Randbereich vertieften Ebene zur Ansammlung von Speichel besonders bevorzugt (vgl. Fig. 8).

Die Sensoreinheit ist besonders bevorzugt als Folie ausgebildet und kann ebenfalls bevorzugt von einer oder mehreren Seiten vorzugsweise vollständig oder zumindest über den Kontaktierungsbereich aller Sensoren mit Speichel benetzt werden.

Die Sensoreinheit kann seitlich oder von oben aufgelegt oder eingeführt werden.

Die elektrischen Kontakte zwischen dem Bürstenkopf und der Sensoreinheit befinden sich vorzugsweise in einem zum Bürstengriff am geringsten beabstandeten Teilbereich einer Auflagefläche für die Sensoreinheit.

Bohrungen sind nicht notwendig, können aber zum Sammeln von Speichel vorgesehen sein.

Ein weiterer Aspekt ist die Erkenntnis, dass der Einfluss von Zahnpasta bzw. Zahnpastaschaum oder ggf. auch Mundwasser den Messvorgang stark stört. Daher sollte die Messung vorteilhaft vor dem eigentlichen Putzvorgang erfolgen. Um die Alltagsroutine nicht zu stark zu beeinflussen kann der Putzvorgang bereits begonnen werden und die Zahnpasta nach der Messzeit, z.B. 5 Sekunden, appliziert werden.

Alternativ oder zusätzlich kann auch eine Messung nach dem Zähneputzen nach der Spülung des Mundraums mit einer Kalibrierspülung erfolgen.

Ein Verfahren zum Betrieb eines Analysensystems umfassend die Zahnbürste und ein externes Gerät zur Datenverarbeitung. Das Gerät kann beispielsweise ein Server, ein Computer, ein Mobiltelefon, ein Tablet und/oder die vorgenannte Austauschstation sein.

Dabei werden Rohsignale, z.B. Impedanzmesswerte, spannungsäquivalente Signale oder dergleichen, von der Zahnbürste auf das externe Gerät übermittelt.

Das externe Gerät weist einen Datenspeicher auf, auf welchem ein Computerprogrammprodukt zur Datenanalyse hinterlegt ist, wobei das Computerprogrammprodukt ausgebildet zur Datenanalyse der von der Sensoreinheit ermittelten Daten, vorzugsweise nach einer oder mehreren DSP-Methoden, zur Ermittlung eines Gesundheitszustandes.

Die besagte Datenanalyse weist, gemäß des Verfahrens, zumindest die folgenden Schritte auf:
a) Abtasten von elektrochemischen Signalen und/oder Impedanzen
b) Detektion und/oder Klassifizierung von Zuständen des Sensorelements hinsichtlich des Befeuchtungszustandes eines Sensorelements bzw. einzelner Sensoren des Befeuchtungselements anhand der ermittelten elektrochemischen Signale und/oder Impedanzen
c) Bewertung von Amplituden und Änderungen der elektrochemischen Signale und/oder Impedanzen um den Speichel hinsichtlich des Gesundheitszustands des Patienten zu analysieren.

Der Befeuchtungszustand kann vollständig oder teilweise (unzureichend) befeuchtet anzeigen. Es kann allerdings auch andere Zustände, wie z.B. die Anwesenheit von Zahnpasta und die Konzentration von Speichel in Wasser angezeigt werden. Dadurch ist es möglich die Konzentration an Speichel zu bestimmen und Störfaktoren wie Zahnpasta bei der Messung und Auswertung des Gesundheitszustands zu kompensieren.

Eine Möglichkeit zur Detektion der vorgenannten Zustände ist die Detektion von Signal-Änderungen, Zeitkonstanten von exponentiellen Faktoren und/oder Signal-Varianz Berechnung (Ripple-Monitoring).

Bei der Bewertung von Amplituden in Schritt c) kann vorteilhaft eine Signalzerlegung der Rohsignale bzw. elektrochemischen Signale zur Analyse des Nutzanteils des jeweiligen Signals und/oder zur Kompensation von Störanteilen erfolgen.

Die Zerlegung kann anhand von exponentiellen Modellen erfolgen, welche z.B. als exponential decays bzw. exponentieller Prozess ausgelegt sein können oder als first order exponential step responses bzw. als Schrittantworten erster Ordnung, insbesondere vom 1-exp(-t) Typ.

Eine weitere Form der Zerlegung kann durch Klassifizierung von Signalen und Signaländerungen basierend auf zeitlichen Mustern (Signalverarbeitung im Zeitbereich) oder auch Merkmalen im Frequenzbereich erfolgen.

Weiterhin ist eine Zerlegung durch Klassifizierung von Signalen basierend auf dem Verlauf von Ableitungen von Signalen und/oder als Änderungsrate bzw. Rate-of-Change und/oder als eine oder mehrere approximative Ableitungen bzw. approximative derivative erfolgen.

Nach der Zerlegung von Signalen in unterschiedliche Komponenten (signal decomposition) kann sodann ein Ableiten von Signalmerkmalen / Charakteristiken basierend auf dieser Zerlegung zur Verbesserung von Schätzungen von Konzentrationen und/oder zum Ableiten von präziseren Kalibrationskurven verwendet werden.

Die Trennung bzw. Separation von unterschiedlichen Störkomponenten vom Nutzanteil des elektrochemischen Signals bzw. der Impedanz, also des Reaktions-Anteil der entsprechenden elektrochemischen Reaktion, kann zudem alternativ oder vorteilhaft mittels der Cottrel-Gleichung oder anderen ähnlichen Modellen physikalischer und/oder mathematischer Natur erfolgen. Das Nutzsignal ist sodann von Interesse ist für die Konzentrationsbestimmung von Indikatoren, insbesondere von Biomarkern, welche einzeln oder ggf. zusammen mit weiteren Messwerten Aussagen über den Gesundheitszustand des Nutzers der Zahnbürste geben können. Die Biomarker können von spezifischen Biosensoren detektiert werden.

Das Verfahren kann zudem eine Abtastung von elektrochemischen Signalen nach einer Konditionierung und/oder Stabilisierung der Sensoreinheit in einer bereitgestellten Referenzlösung aufweisen. Diese Abtastung kann vorzugsweise nach Schritt c) z.B. bei erstmaliger Inbetriebnahme der Zahnbürste erfolgen, oder aber vor Schritt a).

Die Abtastung von Signalen nach erfolgter Konditionierung und/oder Stabilisierung kann vorteilhaft in Kombination durch einen Trigger bzw. Sensor, vorzugsweise einem Sensor zur Leitfähigkeits- oder Impedanzmessung, initiiert oder erkannt werden, welcher die Anwesenheit der Referenzlösung und/oder von Speichel detektiert und/oder das Ende eines Konditionierungszeitraums anzeigt. Der Trigger kann die Messung vorzugsweise basierend auf dem detektierten Zustand oder Zustands-Übergang vornehmen.

Weiterhin kann insbesondere im Anschluss an Schritt c) eine oder mehrere Wiederholungen von Messungen erfolgen, um die Information aus mehreren Messungen zu kombinieren, vorzugsweise um die Präzision zu verbessern, das Signal-zu-Rauschen (S/N signal-to-noise) Verhältnis zum Signal hin zu verbessern und/oder Störungen zu kompensieren.

Das Abtasten von elektrochemischen Signalen, insbesondere von amperometrischen Signalen bei unterschiedlichen Potenzialen kann über mehrere Sekunden oder Minuten erfolgen.

Die Detektion und Klassifizierung von Zuständen "Trocken" vs. "belüftet nach Befeuchtung" vs. "vollständig befeuchtet" Zuständen und den zugehörigen Übergängen (Transitionen) kann wie vorbeschrieben erfolgen.

Diese Detektion ermöglicht vorteilhaft eine Einschätzung über die Zuverlässigkeit der vom Biosensor und den weiteren Sensoren durchgeführten Messungen und dem daraus ermittelten Gesundheitszustand.

Die Bewertung vom Abklingen von elektrochemischen Signalen oder Impedanzen lässt Rückschlüsse auf Redox-Reaktionen und kapazitive Transienten innerhalb des Mundraumes zu.

Die Zahnbürste, insbesondere die Sensoranordnung kann, wie zuvor beschrieben, einen Temperatursensor aufweisen. Die Temperatur-Messung kann über mehrere Tage gemittelt erfolgen, um eine Referenz der Körpertemperatur zu erhalten. Darüber hinaus kann eine sprunghafte Temperaturerhöhung, z.B. bei Fieber, detektiert werden sowie eine auch vorteilhaft allmählich ansteigende und/oder periodisch wiederkehrende Temperaturerhöhung. Dies kann z.B. vorteilhaft für die Bestimmung vom Fertilitäts-Zyklus sein, z.B. als Teil einer Gesundheitsüberwachung bzw. Health Monitoring oder zusätzlich dazu.

Optional oder zusätzlich kann die Zahnbürste eine Dosiervorrichtung zur Abgabe von Stoffen zur Behandlung von Erkrankungen, insbesondere im Mundraum, und/oder zur Stimulation des Immunsystems aufweisen.

Diese können speziell von einem Arzt verschrieben sein, wobei die Zahnbürste als Dosierungsvorrichtung dient. Insbesondere bei Erkrankungen im oralen Bereich, wie z.B. bei Zahnfleischentzündungen, kann durch die Zahnbürste eine ortsgenaue Applikation eines entsprechenden Medikaments und verbunden mit einer mechanisch-unterstützenden Massage während der Dosage durch die Borsten, z.B. am Zahnfleisch, erfolgen. Die Dosierung bzw. den Umfang der Abgabe des Medikaments kann der Arzt oder der Patient an der Zahnbürste vornehmen. Im Fall des Arztes kann dieser über eine spezielle Zugangsberechtigung für entsprechende Unterprogramme, z.B. über einen Bluetooth-Transponder oder einen Code oder dergleichen, verfügen, welcher den Zugriff auf arztspezifische Unterprogramme der Zahnbürste verhindert. Alternativ oder zusätzlich kann auch ein Programm bzw. eine Programmeinstellung zur Dosage mittels Fernzugriff oder über ein Datenspeicher, z.B. USB-Stick, auf die Zahnbürste oder die Austauschstation übertragen werden.

Weiterhin optional kann eine Referenzflüssigkeit zur Konditionierung der Sensoren eingesetzt werden. Hierfür kann sich u.a. Wasser, z.B. Wasser mit einem definierten Ionengehalt, anbieten. Diese Referenzflüssigkeit, auch Konditionierungsflüssigkeit genannt, setzt die Sensoren der Sensoranordnung auf einen Ausgangswert zurück.

Zudem kann eine oder mehrere Kalibrierungsflüssigkeiten eingesetzt werden mit einer oder mehreren gegenüber der Referenzflüssigkeit unterschiedlich-definierten Leitfähigkeiten. Somit können nach einem gewissen Zeitraum die Messgenauigkeit durch die Kalibrationsflüssigkeit bzw. -flüssigkeiten überprüft und ggf. neu eingestellt werden.

Die Referenzflüssigkeit und die zumindest eine Kalibrationsflüssigkeit kann Teil des Analysensystems sein.

Die Sensoranordnung als auch der Bürstenkopf können austauschbar und als Wegwerfartikel (disposable) ausgebildet sein. Die Sensoranordnung erlaubt vorteilhaft sowohl eine Erkennung eines Gesundheitszustands als auch eine Überwachung desselben. In ausgesuchten Fällen einer Erkrankung kann zudem die Art der Krankheit und/oder deren Ausprägung bzw. Intensität festgestellt werden.

Multidimensionale Biomarker können eingesetzt werden. Die Handhabung der Zahnbürste sollte idealerweise derart erfolgen, dass deren Signale zumindest einmal täglich gesammelt und/oder aufgenommen werden
Die Daten können dann über mehrere Tage, Wochen Monate, usw. gespeichert werden, um Langzeittendenzen zu erkennen.

### Bezugszeichenliste

- 1: Bürstenkopf
- 2: Borsten
- 3: Sensoranordnung
- 3': verbrauchte Sensoranordnung
- 4: Zweite Sensoreinheit
- 5: Erste Sensoreinheit
- 6: Elektrode
- 7: Elektrode
- 8: Drittes Sensoreinheit
- 9: Mechanische Schnittstelle
- 10: Austauschstation
- 11: Austauschkammer
- 12: Vereinzelungsvorrichtung
- 13: Folienrolle
- 14: Ladestation
- 15: Auslese- und /oder Auswerteeinheit
- 16: Sendemodul
- 17: Reservoir

- 20: Zahnbürstenkopf
- 21: Einführungsschlitz
- 22: Abdecksegment
- 23: Grundkörper
- 24: Borsten
- 25: Bürstenhals-Ansatz
- 26: Stirnfläche

- 30: Bürstenkopf
- 31: Grundkörper
- 32: Borsten
- 33: Auflagenfläche
- 34: Sensoraufnahme
- 35: Sensoraufnahme
- 36: Sensoraufnahme
- 37: Rand
- 40: Zahnbürstenkopf
- 41: Grundkörper
- 42: Sensoreinheit
- 43: Einführungsschlitz
- 45: Öffnungen
- 47: Abdecksegment
- 50: Bürstenkopf
- 51: Sensoreinheit
- 52: Vorsprung
- 53: Sensorarray
- 54: Vertiefung
- 55: Sensoren

- l_{b}: Borstenlänge
- l: Länge Einführungsschlitz
- h: Höhe Einführungsschlitz
- R: Einsteckrichtung

- 100: Zahnbürste
- 101: Griff

## Patentansprüche

1. Analysensystem umfassend eine Zahnbürste (100) mit einem Bürstenkopf (1, 20, 30, 40, 50), mit zumindest einem mechanischen Reinigungsmittel zur Reinigung der Zähne und mehreren Sensoreinheiten (4, 5, 8, 42, 51) zur Überwachung des Gesundheitszustandes eines Anwenders, wobei der Bürstenkopf (1, 20, 30, 40, 50) eine erste Sensoreinheit (5) aufweist zur Durchführung einer Impedanzmessung des Speichels mit einer oder mehreren Frequenzen und/oder Frequenzbändern,
wobei der Bürstenkopf (1, 20, 30, 40, 50) zumindest eine zweite Sensoreinheil (4) zur Bestimmung des pH-Wertes und/oder der Temperatur von Speichel, aufweist, und
wobei der Bürstenkopf (1) eine dritte Sensoreinheit (8) aufweist, wobei die dritte Sensoreinheit (8) als Biosensor ausgebildet ist und
wobei die dritte Sensoreinheit (8) austauschbar am oder im Bürstenkopf (1, 20, 30, 40, 50) angeordnet ist
und ein externes Gerät zur Datenverarbeitung, wobei die Zahnbürste ein Funkmodul zur Datenübertragung mit dem externen Gerät zur Datenverarbeitung unter Ermittlung eines Gesundheitszustandes aufweist, so dass Rohsignale von der Zahnbürste auf das externe Gerät übermittelbar sind und wobei das externe Gerät einen Datenspeicher aufweist, auf welchem ein Computerprogrammprodukt zur Datenanalyse hinterlegt ist, wobei das Computerprogrammprodukt ausgebildet zur Datenanalyse der von der als Biosensor ausgebildeten Sensoreinheit ermittelten Daten, vorzugsweise nach einer oder mehreren DSP-Methoden, zur Ermittlung eines Gesundheitszustandes,
wobei die Datenanalyse die folgenden Schritte aufweist:
a) Abtasten von Impedanzen über eine Messdauer mit einer vorbestimmten Abtastrate
b) Detektion und Klassifizierung von Zuständen der Sensoreinheiten (4,5,8,42,51) hinsichtlich des Befeuchtungszustandes der Sensoreinheiten (4,5,8,42,51) anhand der ermittelten Impedanzen; und
c) Bewertung von Amplituden und/oder Änderungen der Impedanzen, und insbesondere der damit einhergehenden Redox-Reaktionen und/oder kapazitiven Transienten, um den Speichel hinsichtlich des Gesundheitszustands des Anwenders zu analysieren wobei anhand der ermittelten Impedanzen zusätzlich zum Befeuchtungszustand die Anwesenheit von Zahnpasta und die Konzentration von Speichel in Wasser detektiert werden.

2. Analysensystem nach Anspruch 1, **dadurch gekennzeichnet, dass** der Bürstenkopf, insbesondere die dritte Sensoreinheit (4, 5, 8, 42, 51) ein Reservoir und/oder eine Zuleitung für Abgabestoffe, z.B. funktionalen Molekülen, Bindestoffe, z.B. Bindeliganden, und/oder Indikatorverbindungen, z.B. Luminophore und/oder Biomarker, insbesondere Fluorophore, aufweist.

3. Analysensystem nach einem der vorhergehenden Ansprüche, **dadurch gekennzeichnet, dass** die erste Sensoreinheit (5) zur Bestimmung eines spannungsäquivalenten Messwertes des Speichels in Form eines Widerstandswerts des Speichels oder eines Leitfähigkeitswerts des Speichels ausgebildet ist.

4. Analysensystem nach einem der vorhergehenden Ansprüche, **dadurch gekennzeichnet, dass** der Biosensor als ein Lactat-Sensor, Glucose-Sensor, IgA-Sensor, IgM-Sensor, IgG-Sensor, CRP-Sensor, IL-6-Sensor, Lysozym-Sensor, Tnf-a Sensor, Alpha-Macroglobulin-Sensor und/oder als ein Sensor zur Detektion von Metaboliten, insbesondere von priodontitis-, paradontitis und/oder kariesverursachenden Bakterien, vorzugsweise von Prevotella intermedia, Porphyromonas gingivalis, Tannerella forsythia, Treponema denticola und/oder den mit aggressiver Parodontitis assoziierten Aggregatibacter actinomycetemcomitans und/oder als ein GABA-Sensor zur Ermittlung einer Konzentration der vorgenannten Verbindungen ausgebildet ist.

5. Analysensystem nach einem der vorhergehenden Ansprüche, **dadurch gekennzeichnet, dass** die Zahnbürste (100) einen Griff (101) und den Bürstenkopf (1, 20, 30, 40, 50), sowie eine mechanische Schnittstelle (9) aufweist zur lösbaren Verbindung des Bürstenkopfes (1, 20, 30, 40, 50) mit dem Griff (101).

6. Analysensystem nach einem der vorhergehenden Ansprüche **dadurch gekennzeichnet, dass** die Zahnbürste (100) eine Steuer- und/oder Auswerteeinheit aufweist, welche ausgerüstet ist zum Erfassen von Daten, insbesondere von Rohdaten, während der Messung des Speichels durch die Sensoreinheit (4, 5, 8, 42, 51) mit einer Abtastrate von 10-1000 Hz.

7. Analysensystem nach Anspruch 6, **dadurch gekennzeichnet, dass** die Steuer- und/oder Auswerteeinheit ausgerüstet ist zum Erfassen von Daten, insbesondere von Rohdaten, während der Messung des Speichels durch die Sensoreinheit (4, 5, 8, 42, 51) in einer Messdauer von zumindest 3 bis 500 Sekunden.

## Claims

1. Analysis system, comprising a toothbrush (100) with a brush head (1, 20, 30, 40, 50), having at least one mechanical cleaning agent for cleaning the teeth and a plurality of sensor units (4, 5, 8, 42, 51) for monitoring the state of health of a user, wherein the brush head (1, 20, 30, 40, 50) has a first sensor unit (5) for performing an impedance measurement of the saliva with one or more frequencies and/or frequency bands,
wherein the brush head (1, 20, 30, 40, 50) has at least one second sensor unit (4) for determining the pH value and/or the temperature of saliva, and
wherein the brush head (1) has a third sensor unit (8), wherein the third sensor unit (8) is designed as a biosensor and
wherein the third sensor unit (8) is arranged in an exchangeable manner on or in the brush head (1, 20, 30, 40, 50)
and has an external device for data processing, wherein the toothbrush comprises a radio module for data transmission to the external device for data processing to determine a state of health, so that raw signals can be transmitted from the toothbrush to the external device, and wherein the external device comprises a data memory on which a computer program product for data analysis is stored, wherein the computer program product is designed for analyzing the data determined by the sensor unit designed as a biosensor, preferably according to one or more DSP methods, for determining a state of health,
wherein the data analysis comprises the following steps:
a. sampling impedances over a measurement period at a predetermined sampling rate;
b. detecting and classifying states of the sensor units (4, 5, 8, 42, 51) with regard to the moisture state of the sensor units (4, 5, 8, 42, 51) on the basis of the impedances determined; and
c. evaluating amplitudes and/or changes in the impedances, and in particular the associated redox reactions and/or capacitive transients, in order to analyze the saliva with regard to the state of health of the user, wherein, on the basis of the impedances determined, the presence of toothpaste and the concentration of saliva in water are detected in addition to the humidification state.

2. Analysis system according to claim 1, **characterized in that** the brush head, in particular the third sensor unit (4, 5, 8, 42, 51), has a reservoir and/or a supply line for dispensing additive substances, e.g. functional molecules, binding agents, e.g. binding ligands, and/or indicator compounds, e.g. luminophores and/or biomarkers, in particular fluorophores.

3. Analysis system according to one of the preceding claims, **characterized in that** the first sensor unit (5) is designed to determine a voltage-equivalent measured value of the saliva in the form of a resistance value of the saliva or a conductivity value of the saliva.

4. Analysis system according to one of the preceding claims, **characterized in that** the biosensor is designed as a lactate sensor, glucose sensor, IgA sensor, IgM sensor, IgG sensor, CRP sensor, IL-6 sensor, lysozyme sensor, TNF-a sensor, alpha-macroglobulin sensor and/or as a sensor for detecting metabolites, in particular bacteria causing periodontitis and/or caries, preferably from Prevotella intermedia, Porphyromonas gingivalis, Tannerella forsythia, Treponema denticola and/or Aggregatibacter actinomycetemcomitans associated with aggressive periodontitis, and/or as a GABA sensor for determining a concentration of the aforementioned compounds.

5. Analysis system according to one of the preceding claims, **characterized in that** the toothbrush (100) has a handle (101) and the brush head (1, 20, 30, 40, 50), as well as a mechanical interface (9) for detachably connecting the brush head (1, 20, 30, 40, 50) to the handle (101).

6. Analysis system according to one of the preceding claims, **characterized in that** the toothbrush (100) has a control and/or evaluation unit, which is equipped to record data, in particular raw data, during the measurement of saliva by the sensor unit (4, 5, 8, 42, 51) at a sampling rate of 10-1000 Hz.

7. Analysis system according to claim 6, **characterized in that** the control and/or evaluation unit is equipped to record data, in particular raw data, during the measurement of saliva by the sensor unit (4, 5, 8, 42, 51) in a measurement period of at least 3 to 500 seconds.

## Revendications

1. Système d'analyse comprenant une brosse à dents (100) avec une tête de brosse à dents (1, 20, 30, 40, 50), avec au moins un produit de nettoyage mécanique pour nettoyer les dents et plusieurs unités de capteur (4, 5, 8, 42, 51) pour surveiller l'état de santé d'un utilisateur, la tête de brosse à dents (1, 20, 30, 40, 50) présentant une première unité de capteur (5) pour effectuer une mesure d'impédance de la salive avec une ou plusieurs fréquences et/ou bandes de fréquence, la tête de brosse à dents (1, 20, 30, 40, 50) présentant au moins une deuxième unité de capteur (4) pour déterminer le pH et/ou la température de la salive, et la tête de brosse à dents (1) présentant une troisième unité de capteur (8), la troisième unité de capteur (8) étant conçue comme un biocapteur et la troisième unité de capteur (8) étant située remplaçable sur ou dans la tête de brosse à dents (1, 20, 30, 40, 50), et un appareil externe pour le traitement de données, la brosse à dents présentant un module radio pour la transmission de données avec l'appareil externe pour le traitement de données en déterminant un état de santé, de sorte que des signaux bruts sont transmissibles de la brosse à dents à l'appareil externe et l'appareil externe présentant une mémoire de données, sur laquelle est déposé un produit de programme informatique pour l'analyse des données, le produit de programme informatique étant conçu pour l'analyse des données déterminées par l'unité de capteur conçue comme biocapteur, de préférence selon une ou plusieurs méthodes DSP, pour déterminer un état de santé, l'analyse des données présentant les étapes suivantes :
a) échantillonnage d'impédances sur une durée de mesure avec un taux d'échantillonnage prédéterminé
b) détection et classification d'états des unités de capteur (4, 5, 8, 42, 51) quant à l'état d'humidification des unités de capteur (4, 5, 8, 42, 51) à l'aide des impédances déterminées ; et
c) évaluation des amplitudes et/ou des modifications des impédances, et en particulier des réactions redox et/ou des transitoires capacitifs qui y sont associés, afin d'analyser la salive quant à l'état de santé de l'utilisateur, la présence de dentifrice et la concentration de salive dans l'eau étant détectées à l'aide des impédances déterminées, en plus de l'état d'humidification.

2. Système d'analyse selon la revendication 1, **caractérisé en ce que** la tête de brosse à dents, en particulier la troisième unité de capteur (4, 5, 8, 42, 51), présente un réservoir et/ou une conduite d'alimentation pour des substances de distribution, par exemple des molécules fonctionnelles, des substances de liaison, par exemple des ligands de liaison, et/ou des composés indicateurs, par exemple des luminophores et/ou des biomarqueurs, en particulier des fluorophores.

3. Système d'analyse selon l'une des revendications précédentes, **caractérisé en ce que** la première unité de capteur (5) est conçue pour déterminer une valeur de mesure équivalente en tension de la salive sous la forme d'une valeur de résistance de la salive ou d'une valeur de conductivité de la salive.

4. Système d'analyse selon l'une des revendications précédentes, **caractérisé en ce que** le biocapteur est conçu comme un capteur de lactate, un capteur de glucose, un capteur d'IgA, un capteur d'IgM, un capteur d'IgG, un capteur de CRP, un capteur d'IL-6, un capteur de lysozyme, un capteur de Tnf-a, un capteur d'alpha-macroglobuline et/ou comme un capteur pour la détection de métabolites, en particulier de bactéries provoquant des périodontites, des parodontites et/ou des caries, de préférence de Prevotella intermedia, Porphyromonas gingivalis, Tannerella forsythia, Treponema denticola et/ou des Aggregatibacter actinomycetemcomitans associés à des parodontites agressives, et/ou comme un capteur GABA pour déterminer une concentration des composés précités.

5. Système d'analyse selon l'une des revendications précédentes, **caractérisé en ce que** la brosse à dents (100) comporte un manche (101) et la tête de brosse à dents (1, 20, 30, 40, 50), ainsi qu'une interface mécanique (9) pour relier de manière amovible la tête de brosse à dents (1, 20, 30, 40, 50) au manche (101).

6. Système d'analyse selon l'une des revendications précédentes, **caractérisé en ce que** la brosse à dents (100) présente une unité de commande et/ou d'évaluation qui est équipée pour saisir des données, en particulier des données brutes, pendant la mesure de la salive par l'unité de capteur (4, 5, 8, 42, 51) avec une fréquence d'échantillonnage de 10-1000 Hz.

7. Système d'analyse selon la revendication 6, **caractérisé en ce que** l'unité de commande et/ou d'évaluation est équipée pour l'enregistrement de données, en particulier de données brutes, pendant la mesure de la salive par l'unité de capteur (4, 5, 8, 42, 51) pendant une durée de mesure d'au moins 3 à 500 secondes.
